(19) Europäisches Patentamt
European Patent Office
Office européen des brevets

(11) EP 0 539 274 B1

(12) **FASCICULE DE BREVET EUROPEEN**

(45) Date de publication et mention de la délivrance du brevet:
**10.04.1996 Bulletin 1996/15**

(51) Int. Cl.$^{6}$: **C07C 45/41**, B01J 23/62, C07C 47/21

(21) Numéro de dépôt: **92402844.2**

(22) Date de dépôt: **16.10.1992**

(54) **Procédé de synthèse d'aldéhydes et de leurs dérivés**

Verfahren zur Herstellung von Aldehyden und von deren Abkömmlingen

Method for the synthesis of aldehydes and of their derivatives

(84) Etats contractants désignés:
**AT BE CH DE DK ES FR GB GR IE IT LI LU NL PT SE**

(30) Priorité: **24.10.1991 FR 9113147**

(43) Date de publication de la demande:
**28.04.1993 Bulletin 1993/17**

(73) Titulaire: **RHONE-POULENC CHIMIE**
**F-92408 Courbevoie Cédex (FR)**

(72) Inventeurs:
- **Ferrero, Rose-Marie**
  **F-69003 Lyon (FR)**
- **Jacquot, Roland**
  **F-69110 Sainte Foy les Lyon (FR)**

(74) Mandataire: **Dutruc-Rosset, Marie-Claude et al**
**RHONE-POULENC CHIMIE,**
**Direction de la Propriété Industrielle,**
**25, Quai Paul Doumer**
**F-92408 Courbevoie Cédex (FR)**

(56) Documents cités:
**US-A- 4 328 373**

- **JOURNAL OF CATALYSIS. vol. 121, no. 1, Janvier 1990, DULUTH, MN US pages 174 - 182 V.M. DESHPANDE ET AL. 'Studies on ruthenium-tin boride catalysts. II. Hydrogenation of fatty acid esters to fatty alcohols.'**
- **CHEMICAL ABSTRACTS, vol. 114, no. 11, 18 Mars 1991, Columbus, Ohio, US; abstract no. 101124w, page 668 ;colonne 1 ; & IN-A-165 539**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

EP 0 539 274 B1

## Description

La présente invention concerne un procédé de synthèse d'aldéhydes. Plus particulièrement, elle concerne un procédé de synthèse d'aldéhydes par réduction en phase vapeur d'acides, d'esters ou de leurs dérivés.

Il est connu dans l'art antérieur de préparer des aldéhydes aromatiques ou aliphatiques saturés par réduction des acides ou esters correspondants au moyen d'un catalyseur choisi parmi les oxydes de cérium, zirconium, uranium, praséodyme et yttrium, à une température comprise entre 350 et 450°C (US 4,328,373).

Il est également connu de préparer des aldéhydes aromatiques par réduction des acides ou esters correspondants en présence d'un catalyseur à base d'oxyde de zirconium contenant un additif choisi par exemple parmi le chrome, le manganèse, le fer, le cobalt ou le zinc, ou parmi certains éléments du groupe III de la classification périodique des éléments, tels que l'aluminium, le scandium, l'yttrium ou le gadolinium (EP 150 961). Ce procédé est conduit à des températures supérieures à 300°C.

Compte tenu des conditions de températures requises pour leur mise en oeuvre, ces procédés ne permettent pas de préparer des aldéhydes à partir d'acides thermosensibles tels que les acides phénols. De même, ces procédés ne permettent pas de préparer des aldéhydes insaturés à partir d'acides insaturés.

La présente invention permet de remédier à ces inconvénients. La demanderesse a en effet montré que les catalyseurs bimétalliques ruthénium-étain permettaient :

- d'hydrogéner des acides à des températures plus douces, ce qui rend possible la réduction d'acides thermosensibles en aldéhydes,
- de réduire sélectivement des acides ou esters insaturés en aldéhydes insaturés, ou encore,
- de préparer avec des rendements élevés des aldéhydes à partir d'acides aromatiques ou aliphatiques saturés.

Un objet de l'invention réside donc dans un procédé de préparation d'aldéhydes par réduction d'acides, d'esters ou d'anhydrides carboxyliques caractérisé en ce que l'on opère en phase vapeur, en présence d'un catalyseur bimétallique de type ruthénium-étain.

Dans l'exposé qui suit de la présente invention, on entend par catalyseur bimétallique de type ruthénium-étain, un catalyseur comprenant comme éléments actifs, au moins du ruthénium et de l'étain.

Des catalyseurs de type Ru/Sn/B ont déjà été décrits dans la littérature [J. Cat., 121, 165-173 (1990)], ainsi que leur utilisation pour la réduction, en phase liquide, d'acides gras saturés et insaturés en alcools gras correspondants [J. Cat., 121, 174-182 (1990)]. Toutefois, rien dans ces documents ne suggère la possibilité d'utiliser des catalyseurs bimétalliques de type ruthénium-étain pour la préparation d'aldéhydes à partir d'acides.

L'invention est plus particulièrement adaptée à la préparation d'aldéhydes de formule générale :

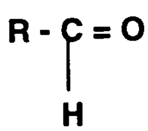
(I)

dans laquelle R représente un atome d'hydrogène ou un radical hydrocarboné, éventuellement substitué comportant de 1 à 40 atomes de carbone qui peut être un radical aliphatique acyclique saturé ou insaturé, linéaire ou ramifié ; un radical carbocyclique ou hétérocyclique saturé, insaturé ou aromatique, monocyclique ou polycyclique, par réduction d'esters, d'anhydrides ou d'acides de formule :

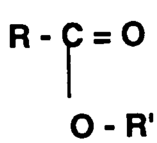
(II)

dans laquelle :

- R est défini comme précédemment,
- R′ représente :
    - un groupement R tel que précédemment défini,

- un groupement

$$R'' - \underset{|}{C} = O$$

dans lequel R″ a la signification donnée pour R,
- les deux groupements R et R″ pouvant être liés entre eux pour former un cycle saturé ou insaturé ayant de 5 à 7 atomes et comprenant la fonction anhydride,
- les deux groupements R et R″ par l'intermédiaire de deux atomes vicinaux pouvant former ensemble un pont d'un système bicyclique ortho-condensé.

Les acides carboxyliques ou dérivés mis en oeuvre préférentiellement répondent à la formule (II) dans laquelle R représente un radical hydrocarboné, éventuellement substitué comportant de 1 à 20 atomes de carbone.

Le procédé de l'invention s'applique à tout acide carboxylique mono- ou polycarboxylique tels que les acides aliphatiques saturés ou insaturés ; carbocycliques ou hétérocycliques, saturés, insaturés ou aromatiques, monocycliques ou polycycliques ; aliphatiques saturés ou insaturés porteurs d'un substituant cyclique tel qu'un cycle carbocyclique ou hétérocyclique saturé, insaturé ou aromatique.

Il convient tout à fait bien, à la préparation d'aldéhydes à partir d'acides carboxyliques aliphatiques saturés tels que le fluoral ou insaturés, notamment ceux qui présentent une double liaison conjuguée avec le groupement carbonyle de la fonction carboxylique, ester ou anhydride.

Il est très bien adapté à la synthèse d'aldéhydes à partir d'acides carboxyliques aromatiques, notamment d'acides benzoïques en particulier les acides hydroxybenzoïques et les acides halogénobenzoïques, de préférence les acides fluorobenzoïques.

Dans l'exposé qui suit de la présente invention, on entend par composé aromatique, la notion classique d'aromaticité telle que définie dans la littérature, notamment par Jerry MARCH - Advanced Organic Chemistry, 3ème édition, John Wiley and Sons, 1985 p. 37 et suivantes.

On entend par acide benzoïque, tout composé aromatique portant au moins une fonction COOH.

Conformément au procédé de l'invention, on peut mettre en oeuvre n'importe quel acide carboxylique susceptible d'être sous forme gazeuse dans les conditions de l'invention.

On peut donc faire appel comme matière première de départ à un acide carboxylique répondant à la formule (II) dans laquelle le reste R représente un radical hydrocarboné, substitué ou non qui peut être un radical aliphatique acyclique saturé ou insaturé, linéaire ou ramifié ; un radical carbocyclique ou hétérocyclique saturé, insaturé ou aromatique, monocyclique ou polycyclique.

Conviennent tout particulièrement bien à la mise en oeuvre du procédé de l'invention, les acides carboxyliques de formule générale (II) dans laquelle R représente un reste hydrocarboné aromatique éventuellement, substitué, monocyclique ou polycyclique.

N'importe quel substituant peut être présent sur le cycle dans la mesure où il ne gêne pas la réaction de réduction de la fonction carboxylique.

R représente préférentiellement un reste hydrocarboné aromatique, et notamment benzénique répondant à la formule générale (III) :

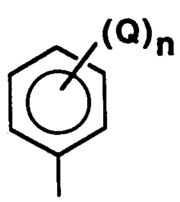

(III)

dans ladite formule (III) :

- n est un nombre entier de 0 à 5, de préférence de 0 à 3,
- Q représente $R_1$, l'un des groupes ou fonctions suivantes :

  . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,

- . un radical alcényle linéaire ou ramifié ayant de 2 à 6 atomes de carbone, de préférence, de 2 à 4 atomes de carbone, tel que vinyle, allyle,
- . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
- . un groupe acyle ayant de 2 à 6 atomes de carbone,
- . un radical de formule :

$$-R_2-OH$$

$$-R_2-COOR_5$$

$$-R_2-CHO$$

$$-R_2-NO_2$$

$$-R_2-CN$$

$$-R_2-(NR_5)_2$$

$$-R_2-CO-(NR_5)_2$$

$$-R_2-SH$$

$$-R_2-X$$

$$-R_2-CF_3$$

dans lesdites formules, $R_2$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; $R_5$ représente un atome d'hydrogène ou un radical alkyle linéaire ou ramifié ayant de 1 à 6 atomes de carbone; X symbolise un atome d'halogène, de préférence un atome de chlore, de brome ou de fluor.

- Q représente $R_3$, l'un des radicaux plus complexes suivants :

- . un radical

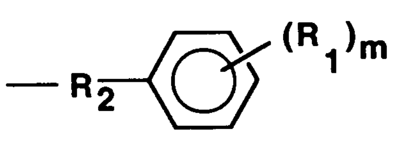

dans lequel $R_1$ et $R_2$ ont la signification donnée précédemment et m est un nombre entier de 0 à 5, de préférence de 0 à 3,

- . un radical $-R_2-A-R_4$ dans lequel $R_2$ a la signification donnée précédemment, $R_4$ représente un radical alkyle linéaire ou ramifié ayant de 1 à 6 atomes de carbone, de préférence, de 1 à 4 atomes de carbone ou un radical

de formule

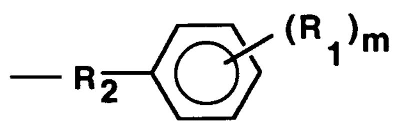

et A symbolise l'un des groupes suivants :

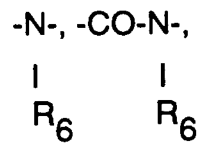

dans ces formules, $R_6$ représente un atome d'hydrogène ou un radical alkyle linéaire ou ramifié ayant de 1 à 4 atomes de carbone, de préférence, un radical méthyle ou éthyle.

Lorsque n est supérieur à 1, les radicaux Q peuvent être identiques ou différents et 2 atomes de carbone successifs du cycle benzénique peuvent être reliés entre eux par un pont cétalique tels que les radicaux méthylène dioxy ou éthylène dioxy extranucléaires.

De préférence, n est égal à 0, 1, 2 ou 3.

Parmi tous les restes R précités, on met en oeuvre tout préférentiellement dans le procédé de l'invention, les acides carboxyliques ou dérivés répondant à la formule générale (II) dans laquelle R représente un reste aromatique répondant à la formule générale (III) dans laquelle :

- n est égal à 0, 1, 2 ou 3,
- Q représente l'un des groupes ou fonctions suivantes :
  - . un atome d'hydrogène,
  - . un radical alkyle linéaire ou ramifié, ayant de 1 à 4 atomes de carbone,
  - . un radical alkoxy linéaire ou ramifié ayant de 1 à 4 atomes de carbone,
  - . un radical méthylène ou éthylène dioxy,
  - . un groupe -OH,
  - . un groupe -CHO,
  - . un groupe $NH_2$,
  - . un radical phényle,
  - . un atome d'halogène,
  - . un groupe $CF_3$.

Encore plus préférentiellement, on choisit les composés de formule (II) dans laquelle les radicaux Q identiques ou différents sont un atome d'hydrogène, un groupe hydroxyle, un radical méthyle, un radical méthoxy, un groupe -CHO.

Comme exemples de radicaux R répondant à la formule (III), on peut mentionner plus précisément les radicaux phényle, tolyle ou xylyle et les radicaux biphényle, méthylène-1,1′ biphényle, isopropylidène-1,1′ biphényle, oxy-1,1′ biphényle, imino-1,1′ biphényle : lesdits radicaux pouvant être substitués par un ou plusieurs radicaux Q tels que prédéfinis, de préférence un groupe hydroxyle ou un atome d'halogène.

R peut également représenter un reste hydrocarboné aromatique polycyclique ; les cycles pouvant former entre eux des systèmes ortho-condensés, ortho- et péri-condensés. On peut citer plus particulièrement, un reste naphtalénique ; lesdits cycles pouvant être substitués par 1 à 4 radicaux $R_1$, de préférence 1 à 3, $R_1$ ayant les significations énoncées précédemment pour les substituants du reste hydrocarboné aromatique de formule générale (III).

Dans la formule générale (II) des acides carboxyliques, R peut représenter également un reste carbocyclique saturé ou comprenant 1 ou 2 insaturations dans le cycle, ayant généralement de 3 à 7 atomes de carbone, de préférence, 6 atomes de carbone dans le cycle ; ledit cycle pouvant être substitué par 1 à 5 radicaux $R_1$, de préférence 1 à 3, $R_1$ ayant les significations énoncées précédemment pour les substituants du reste hydrocarboné aromatique de formule générale (III).

Comme exemples préférés de radicaux R, on peut citer les radicaux cyclohexyle ou cyclohexène-yle, éventuellement substitué par des radicaux alkyle linéaires ou ramifiés, ayant de 1 à 4 atomes de carbone.

Comme mentionné précédemment, R peut représenter un reste aliphatique acyclique, saturé ou insaturé, linéaire ou ramifié.

Plus précisément, R représente un reste aliphatique acyclique linéaire ou ramifié ayant de préférence de 1 à 12 atomes de carbone, saturé ou comprenant une à plusieurs insaturations sur la chaîne, généralement, 1 à 3 insaturations qui peuvent être des doubles liaisons simples ou conjuguées ou des triples liaisons.

La chaîne hydrocarbonée peut être éventuellement :

- interrompue par l'un des groupes suivants :
  -O-, -CO-,

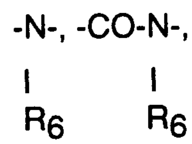

  -S-, $-SO_2-$
  dans ces formules $R_6$ représente l'hydrogène ou un radical alkyle linéaire ou ramifié ayant de 1 à 4 atomes de carbone, de préférence, un radical méthyle ou éthyle,
- et/ou porteuse de l'un des substituants suivants :
  -OH, $-COOR_5$, -CHO, $-NO_2$, -CN, $-NH_2$, -SH, -X, $-CF_3$,
  dans ces formules, $R_5$ ayant la signification donnée précédemment.

Dans un mode préféré de l'invention, R répond à la formule suivante :

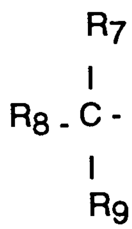
(IV)

dans laquelle $R_7$, $R_8$ et $R_9$, identiques ou différents, sont choisis parmi un atome d'hydrogène, un radical alkyle linéaire ou ramifié contenant 1 à 10 atomes de carbone, un radical alcényle linéaire ou ramifié contenant 1 à 10 atomes de carbone,un radical alkoxy linéaire ou ramifié contenant 1 à 10 atomes de carbone, un groupe hydroxyle, une fonction amine ou un atome d'halogène ou un groupe $-CF_3$.

Préférentiellement, $R_7$ et/ou $R_8$ et/ou $R_9$ représentent un groupement insaturé.

Encore plus préférentiellement, dans la formule (IV), l'un des 3 groupements $R_7$, $R_8$ et $R_9$ possède une double liaison conjuguée avec le groupement carbonyle de l'acide, de l'ester ou de l'anhydride carboxylique.

Il est également possible de faire appel à un acide carboxylique ou dérivé de formule (II) dans laquelle R représente un reste aliphatique acyclique, saturé ou insaturé, linéaire ou ramifié pouvant être éventuellement porteur d'un substituant cyclique. Par cycle, on entend un cycle carbocyclique ou hétérocyclique, saturé, insaturé ou aromatique.

Le reste aliphatique acyclique peut être relié au cycle par un lien valentiel ou par l'un des groupes suivants :
-O-, -CO-,

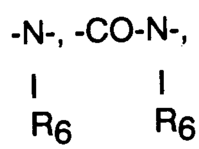

-S-, $-SO_2-$
dans ces formules, $R_6$ ayant la signification donnée précédemment.

Comme exemples de substituants cycliques, on peut envisager des substituants cycloaliphatiques, aromatiques ou hétérocycliques, notamment cycloaliphatiques comprenant 6 atomes de carbone dans le cycle ou benzéniques, ces substituants cycliques étant eux-mêmes éventuellement porteurs de 1, 2, 3, 4 ou 5 radicaux $R_1$, identiques ou différents, $R_1$ ayant les significations énoncées précédemment pour les substituants du reste hydrocarboné aromatique de formule générale (III).

Comme exemples de tels radicaux, on peut mentionner, entre autres, le radical benzyle.

Dans la formule générale (II) des acides carboxyliques, R peut également représenter un reste hétérocyclique, saturé ou non, comportant notamment 5 ou 6 atomes dans le cycle dont 1 ou 2 hétéroatomes tels que les atomes d'azote, de soufre et d'oxygène ; les atomes de carbone de l'hétérocycle pouvant éventuellement être substitués, dans leur totalité ou pour une partie d'entre eux seulement par des radicaux $R_1$, $R_1$ ayant les significations énoncées précédemment pour les substituants du reste hydrocarboné aromatique de formule générale (III).

R peut aussi représenter un reste hétérocyclique polycyclique défini comme étant soit un radical constitué par au moins 2 hétérocycles aromatiques ou non contenant au moins un hétéroatome dans chaque cycle et formant entre eux des systèmes ortho- ou ortho- et péri-condensés ou soit un radical constitué par au moins un cycle hydrocarboné aromatique ou non et au moins un hétérocycle aromatique ou non formant entre eux des systèmes ortho- ou ortho- et péricondensés; les atomes de carbone desdits cycles pouvant éventuellement être substitués, dans leur totalité ou pour une partie d'entre eux seulement par des radicaux $R_1$, $R_1$ ayant les significations énoncées précédemment pour les substituants du reste hydrocarboné aromatique de formule générale (III).

A titre d'exemples de groupements R de type hétérocyclique, on peut citer entre autres, les radicaux furyle, pyrrolyle, thiényle, isoxazolyle, furazannyle, isothiazolyle, imidazolyle, pyrazolyle, pyridyle, pyridazinyle, pyrimidinyle et les radicaux quinolyle, naphtyridinyle, benzofurannyle, indolyle.

A titre d'acides carboxyliques comprenant au moins un groupe carboxylique répondant à la formule (II), on fait appel plus particulièrement aux acides carboxyliques suivants :

- les acides monocarboxyliques aliphatiques saturés tels que l'acide formique, acétique, propionique, butyrique, isobutyrique, valérique, isovalérique, pivalique, laurique, myristique, palmitique, stéarique,
- les acides dicarboxyliques aliphatiques saturés tels que l'acide oxalique, malonique, succinique, glutarique, adipique, pimélique, subérique, azélaïque, sébacique,
- les acides monocarboxyliques ou dicarboxyliques aliphatiques insaturés tels que l'acide acrylique, propiolique, méthacrylique, crotonique, isocrotonique, sénécioïque, oléïque, maléique, fumarique, citraconique, mésaconique,
- les acides carboxyliques carbocycliques saturés ou insaturés tels que l'acide camphorique, l'acide chrysanthémique,
- les acides carboxyliques hétérocycliques tels que les acides furannecarboxyliques, thiophènecarboxyliques, pyrrolecarboxyliques, pyrazinecarboxyliques, l'acide nicotinique, isonicotinique, l'acide picolinique,
- les acides carboxyliques carbocycliques aromatiques tels que l'acide benzoïque, phtalique, isophtalique, téréphtalique, les acides naphtalènecarboxyliques, les acides toluiques,
- les acides carboxyliques arylaliphatiques saturés tels que, notamment les arylpropioniques comme l'acide phényl-2 propionique, l'acide [(butyl-2)-4 phényl]-2 propionique, l'acide (benzoyl-3 phényl)-2 propionique, l'acide (méthoxy-6 naphtyl-2)-2 propionique ou les acides insaturés comme par exemple l'acide phényl-2 propénoïque, l'acide cinnamique,
- les acides carboxyliques aliphatiques ou aromatiques halogénés tels que l'acide monofluoroacétique, difluroacétique, monochloroacétique, dichloroacétique, trichloroacétique, monochloropropionique, α-bromopropionique, α-bromobutyrique, trifluoroacétique, l'acide monofluoro-o-benzoïque, l'acide monofluoro-m-benzoïque, l'acide monofluoro-p-benzoïque, l'acide difluoro-2,3 benzoïque, l'acide difluoro-2,4 benzoïque, l'acide difluoro-2,5 benzoïque, l'acide difluoro-3,4 benzoïque, l'acide trifluoro-2,3,6 benzoïque, l'acide trifluoro-2,4,5 benzoïque, l'acide tétrafluoro-2,3,4,5 benzoïque, l'acide pentafluorobenzoïque, l'acide α,α,α-trifluoro-o-toluique, l'acide α,α,α-trifluoro-m-toluique, l'acide α,α,α-trifluoro-p-toluique, l'acide monochloro-o-benzoïque, l'acide monochloro-m-benzoïque, l'acide monochloro-p-benzoïque, l'acide dichloro-2,3 benzoïque, l'acide dichloro-2,4 benzoïque, l'acide dichloro-2,5 benzoïque, l'acide dichloro-2,6 benzoïque, l'acide dichloro-3,4 benzoïque, l'acide dichloro-3,5 benzoïque, l'acide trichloro-2,3,5 benzoïque, l'acide trichloro-2,3,6 benzoïque, l'acide chloro-2 fluoro-4,5 benzoïque, l'acide chloro-3 trifluro-2,4,5 benzoïque, l'acide monobromo-o-benzoïque, l'acide monobromo-m-benzoïque, l'acide monobromo-p-benzoïque.
- les hydroxy-acides aliphatiques, cycloaliphatiques, arylaliphatiques, tels que l'acide glycolique, l'acide lactique, l'acide glycérique, l'acide hydroxy-2 butanoïque, l'acide hydroxy-3 butanoïque, l'acide méthyl-2 lactique, l'acide hydroxy-2 méthylthio-4 butanoïque, l'acide tartronique, l'acide malique, l'acide tartrique, l'acide hydroxy-1 cyclopropane carboxylique, l'acide hydroxy-2 phénylpropanoïque, l'acide hydroxy-2 cinnamique, l'acide hydroxy-3 cinnamique, l'acide hydroxy-4 cinnamique,
- les acides hydroxybenzoïques suivants : l'acide hydroxy-2 benzoïque (acide salicylique), l'acide hydroxy-3 benzoïque, l'acide hydroxy-4 benzoïque, l'acide méthyl-3 salicylique, l'acide méthyl-4 salicylique, l'acide méthyl-5 salicylique, l'acide hydroxy-3 méthyl-4 benzoïque, l'acide méthoxy-3 salicylique, l'acide méthoxy-4 salicylique, l'acide méthoxy-5 salicylique, l'acide hydroxy-3 méthoxy-4 benzoïque (acide isovanillique), l'acide hydroxy-4 méthoxy-3 benzoïque (acide vanillique), l'acide hydroxy-3 diméthoxy-4,5 benzoïque, l'acide hydroxy-4 diméthoxy-3,5 benzoïque (acide syringique), l'acide hydroxy-5 isophtalique, l'acide amino-3 salicylique, l'acide amino-4 salicylique, l'acide amino-5 salicylique, l'acide hydroxy-3 amino-2 benzoïque, l'acide nitro-3 salicylique, l'acide hydroxy-3 nitro-4 benzoïque, l'acide hydroxy-4 nitro-3 benzoïque, l'acide hydroxy-3 méthyl-4 nitro-2 benzoïque, l'acide diiodo-3,5 salicylique, l'acide dihydroxy-2,3 benzoïque, l'acide dihydroxy-2,4 benzoïque, l'acide dihydroxy-2,5 benzoïque, l'acide dihydroxy-2,6 benzoïque, l'acide dihydroxy-3,4 benzoïque (acide protocatéchique), l'acide dihydroxy-3,5 benzoïque, l'acide dihydroxy-3,5 méthyl-4 benzoïque, l'acide trihydroxy-2,3,4 benzoïque, l'acide trihydroxy-2,4,6 benzoïque, l'acide trihydroxy-3,4,5 benzoïque,

- les alcoxy- et phénoxyacides tels que l'acide méthoxyacétique, phénoxyacétique, dichloro-2,4 phénoxyacétique, phénoxypropionique, dichloro-2,4 phénoxypropionique, p-hydroxyphénoxypropionique, m-chlorophénoxypropionique, l'acide phénoxy-4 benzoïque, l'acide (carboxy-4 phénoxy-4) benzoïque, l'acide pipéronylique,
- les oxo-acides tels que l'acide acétyl-2 benzoïque, l'acide acétyl-4 benzoïque, l'acide benzoyl-2 benzoïque, l'acide benzoyl-4 benzoïque,
- les acyloxy-acides tels que l'acide benzoyloxy-3 propionique, l'acide acétoxy-2 benzoïque, l'acide acétoxy-4 benzoïque,
- les amido-acides tels que l'acide acétamido-2 acrylique, l'acide acétamido-2 benzoïque, l'acide acétamido-3 benzoïque, l'acide N-acétamido-4 benzoïque,
- les acides aminés éventuellement N- protégés par un groupe protecteur comme par exemple les groupes suivants acyle (acétyle, benzoyle), BOC (butyloxycarbonyl), CBZ (carbobenzoxy), FMOC (fluorényl-9 méthoxycarbonyl), MSOC (méthanesulfényl-2 éthoxycarbonyl).

On peut citer les acides aminés suivants :

- acides aminés aliphatiques : glycine, alanine, valine, leucine, isoleucine,
- acides aminés hydroxylés : sérine, thréonine,
- acides aminés soufrés : cystéine, méthionine,
- acides aminés dicarboxyliques et leurs amides : acide aspartique, asparagine, acide glutamique, glutamine,

  . acides aminés possédant deux groupements basiques : lysine, arginine, histidine,
  . acides aminés aromatiques : phénylalanine, tyrosine, tryptophanne,
  . imino-acides : proline, hydroxyproline.

Parmi tous les composés cités précédemment à titre illustratif et sans caractère limitatif, le procédé de l'invention s'applique particulièrement bien aux composés suivants :

- l'acide salicylique et l'acide hydroxy-4 benzoïque.
- l'acide acétique, l'acide propionique et leurs dérivés substitués par un groupe hydroxy, halogène, phényle, phényloxy,
- l'acide benzoïque et ses dérivés substitués par un groupe alkyle $C_1$-$C_4$, acétoxy, acétamido, hydroxy, méthoxy, éthoxy,
- les acides carboxyliques aliphatiques ou aromatiques halogénés tels que l'acide monofluoroacétique, difluroacétique, monochloroacétique, dichloroacétique, trichloroacétique, monochloropropionique, α-bromopropionique, α-bromobutyrique, trifluoroacétique, l'acide monofluoro-o-benzoïque, l'acide monofluoro-m-benzoïque, l'acide monofluoro-p-benzoïque, l'acide difluoro-2,3 benzoïque, l'acide difluoro-2,4 benzoïque, l'acide difluoro-2,5 benzoïque, l'acide difluoro-3,4 benzoïque, l'acide trifluoro-2,3,6 benzoïque, l'acide trifluoro-2,4,5 benzoïque, l'acide tétrafluoro-2,3,4,5 benzoïque, l'acide pentafluorobenzoïque, l'acide α,α,α-trifluoro-o-toluique, l'acide α,α,α-trifluoro-m-toluique, l'acide α,α,α-trifluoro-p-toluique, l'acide monochloro-o-benzoïque, l'acide monochloro-m-benzoïque, l'acide monochloro-p-benzoïque, l'acide dichloro-2,3 benzoïque, l'acide dichloro-2,4 benzoïque, l'acide dichloro-2,5 benzoïque, l'acide dichloro-2,6 benzoïque, l'acide dichloro-3,4 benzoïque, l'acide dichloro-3,5 benzoïque, l'acide trichloro-2,3,5 benzoïque, l'acide trichloro-2,3,6 benzoïque, l'acide chloro-2 fluoro-4,5 benzoïque, l'acide chloro-3 trifluro-2,4,5 benzoïque, l'acide monobromo-o-benzoïque, l'acide monobromo-m-benzoïque, l'acide monobromo-p-benzoïque.
- l'acide nicotinique.

Comme indiqué précédemment, il est également possible de mettre en oeuvre l'acide carboxylique tel que défini ci-dessus sous la forme de son ester. Dans ce cas, dans la formule (II), R′ représente de préférence un radical aliphatique contenant de 1 à 10 atomes de carbone, éventuellement substitué. Plus préférentiellement, R′ représente un radical alkyle linéaire ou ramifié ayant de 1 à 6 atomes de carbone. A titre d'exemples de radicaux R′ préférés, on peut citer les radicaux méthyle, éthyle ou hexyle.

Les esters préférés sont ceux qui dérivent de la liste des acides carboxyliques précités.

Conformément à la présente invention, on peut faire appel à un acide carboxylique sous la forme de son anhydride.

Comme exemples d'anhydrides carboxyliques, on peut mentionner plus particulièrement les anhydrides des acides carboxyliques précités et les anhydrides cycliques.

En effet, lorsque l'anhydride répond à la formule (II) dans laquelle R′ est un groupement

$$R'' - C = O,$$

|

les deux groupements R et R″ peuvent être liés entre eux pour former un cycle saturé ou insaturé ayant de 5 à 7 atomes comprenant la fonction anhydride. Ils forment de préférence un radical alkylène linéaire ou ramifié ayant de 2 à 6 atomes de carbone et encore plus préférentiellement un radical $-(CH_2)_t-$ avec t égal de 2 à 4.

Comme exemples de tels anhydrides cycliques, on peut citer l'anhydride succinique ou l'anhydride maléique.

Lorsque l'anhydride répond à la formule (II) dans laquelle R′ est un groupement

$$R'' - C = O,$$

|

les deux groupements R et R″, par l'intermédiaire de deux atomes vicinaux peuvent former ensemble un pont d'un système bicyclique orthocondensé.

Les composés préférés sont bicycliques et constitués d'un cycle benzénique et d'un hétérocycle puisque le cycle comprend l'atome d'oxygène de la fonction anhydride, ledit cycle ayant de préférence de 5 à 6 atomes. Comme exemples de tels anhydrides cycliques d'acides polycarboxyliques, on peut mentionner l'anhydride phtalique.

Le procédé de l'invention est mis en oeuvre en phase gazeuse. Avantageusement, la réaction est conduite à une température comprise entre 150°C et 500°C, et encore plus préférentiellement entre 200 et 400°C. Il est entendu que la température est adaptée par l'homme de l'art en fonction de l'acide de départ, et de la vitesse de réaction recherchée.

Par ailleurs, il peut être particulièrement avantageux de procéder à une activation préalable du catalyseur, par forte élévation de température. Notamment, le catalyseur peut être préalablement soumis à des températures proches de 500°C environ, et préférentiellement de 450°C. L'activation est conduite avantageusement sous courant d'hydrogène.

Une manière pratique de mettre en oeuvre la présente invention consiste à introduire dans un réacteur une quantité désirée de catalyseur, éventuellement entre 2 lits de quartz pour favoriser la mise en contact des réactifs. La température du réacteur est ensuite élevée sous courant d'hydrogène jusqu'à une valeur déterminée, permettant d'activer le catalyseur, puis ramenée à la température de réaction. L'acide est ensuite injecté au débit souhaité et l'aldéhyde formé est récupéré.

Préférentiellement, l'acide est injecté directement sous forme gazeuse après avoir été vaporisé par chauffage.

Toutefois, il peut également être injecté en solution dans un solvant inerte pour la réaction. On peut citer en particulier comme solvants inertes les hydrocarbures aliphatiques (par exemple l'hexane), alicycliques (par exemple le cyclohexane), aromatiques (par exemple le toluène), ou les éthers (par exemple le diméthoxyéthane).

Sous l'effet de la température élevée, l'acide ainsi injecté est vaporisé au niveau du premier lit de quartz. L'hydrogène peut être injecté à la pression atmosphérique ou sous une légère pression compatible avec la phase vapeur (quelques bar, par exemple de 5 à 10 bar). L'hydrogène peut également être dilué dans un gaz inerte tel que l'azote ou l'hélium.

Avantageusement, pour 1 ml de catalyseur, l'hydrogène est injecté à un débit compris entre 0,1 et 10 litres par heure, et l'acide à un débit liquide au plus égal à 10 ml/h, et de préférence compris entre 0,5 et 5 ml/h.

En fin de réaction, on récupère l'aldéhyde par tout moyen approprié tel que distillation ou cristallisation. Dans certains cas, notamment dans le cas du fluoral, l'aldéhyde peut être obtenu sous une forme hydratée.

Différents types de catalyseurs peuvent être utilisés dans la présente invention. En particulier, le catalyseur utilisé peut être supporté ou non.

Plus généralement, le ruthénium représente entre 0,1 et 50 % du poids du catalyseur.

Dans le cas où l'on fait appel à un catalyseur massique, le ruthénium représente de 10 % à 50 % du poids du catalyseur.

Dans un mode préféré de mise en oeuvre, on utilise toutefois un catalyseur sous forme supportée. A cet effet, le support peut être choisi parmi les oxydes de métaux, tels que les oxydes d'aluminium, de silicium et/ou de zirconium, ou encore parmi les charbons éventuellement activés par un traitement bien connu à l'acide nitrique, le noir d'acétylène, ou les résines. Il est généralement préférable de choisir un support n'ayant pas une surface spécifique trop élevée, pour limiter les risques d'interaction directe du support avec les réactifs de la réaction.

Dans le cas d'un catalyseur supporté, la teneur en ruthénium est adaptée par l'homme de l'art en fonction du support (nature, surface spécifique) et de la vitesse de réaction recherchée.

Généralement, la teneur en ruthénium du catalyseur est avantageusement choisie entre 0,1 et 20,0 % en poids, et encore plus préférentiellement, entre 0,5 et 3,0 % en poids.

Précisons uniquement à titre d'exemples, que la surface spécifique (B.E.T.) du support est avantageusement choisie entre 50 et 100 $m^2/g$ lorsque la teneur en ruthénium du catalyseur est comprise entre 0,1 et 1 %.

Avantageusement, on utilise dans l'invention des catalyseurs dans lesquels le rapport molaire entre l'étain et le ruthénium engagés étain (Sn)/ruthénium (Ru) est compris entre 1 et 10 inclus, et, encore plus préférentiellement, entre 2 et 6 inclus.

Un premier mode de réalisation de l'invention consiste à faire appel à un catalyseur ruthénium-étain-bore.

Dans ce cas, la teneur en bore est généralement inférieure à 1 % et le plus souvent voisine de 0,5 %.

Lesdits catalyseurs utilisables dans le procédé de l'invention peuvent être préparés de différentes manières et notamment par imprégnation selon le procédé décrit par Desphande et al. [J. Cat., 121, 165-173 (1990)].

Généralement, on dissout dans l'eau les deux métaux sous forme de sels, éventuellement en présence du support, et on laisse l'imprégnation s'effectuer sur une période de 15 heures environ. Selon les cas, on peut ensuite ajouter un réducteur chimique, avant de récupérer et laver le catalyseur bimétallique. Celui-ci est ensuite séché à l'air avant son emploi. Parmi les réducteurs chimiques utilisables, on peut citer notamment les borohydrures, tels que le borohydrure de sodium, le borohydrure de lithium, le borohydrure de potassium ou le borohydrure de tétrabutylammonium; l'hydrazine ou encore le formol.

Un autre mode préféré du procédé de l'invention, consiste à avoir recours à un nouveau catalyseur bimétallique comprenant du ruthénium et de l'étain, exempt de bore, les éléments actifs étant de préférence supportés.

Pour le préparer, on peut recourir à des techniques classiques, connues en elles-mêmes, de préparation de catalyseurs métalliques supportés.

L'un de ses procédés de préparation consiste, par exemple, à introduire un support dans une solution que l'on prépare en dissolvant au moins un composé approprié des éléments choisis ; le dépôt des éléments actifs sur le support est réalisé en distillant le solvant de préférence l'eau qui peut êtr éliminée par évaporation sous pression réduite choisie de préférence entre 5 et 20 mm de mercure. La masse de contact ainsi obtenue est soumise à une réduction au moyen d'un courant d'hydrogène.

Selon un autre mode de préparation classique, le dépôt du ou des composés apportant les éléments métalliques sur le support est réalisé en précipitant les composés de manière en soi connue et en soumettant la masse de contact ainsi obtenue à une réduction au moyen de l'hydrogène.

Le dépôt sur le support de plusieurs éléments métalliques peut bien entendu être réalisé successivement mais de préférence simultanément.

La nature des composés apportant les éléments métalliques utilisés pour la préparation des catalyseurs de l'invention n'est pas critique.

On peut faire appel aux métaux eux-mêmes tels que le ruthénium et l'étain.

A titre d'exemples de composés susceptibles d'être mis en oeuvre pour la préparation des catalyseurs de l'invention, on peut citer, à titre de composés du ruthénium, le chlorure de ruthénium III, le chlorure de ruthénium IV, le pentafluorure de ruthénium, l'oxyde de ruthénium II, l'oxyde de ruthénium IV, l'oxychlorure de ruthénium ammoniaqué $Ru_2(OH)_2Cl_4$, $7NH_3$, $5H_2O$, l'acétate de ruthénium et, à titre de composés de l'étain, les oxydes, chlorures, nitrates, carboxylates, alcoolates d'étain ou des composés organométalliques dans lesquels l'étain est lié à un atome d'hydrogène et/ou des radicaux alkyle ayant de préférence de 1 à 4 atomes de carbone. Les sels préférés sont les suivants : les composés du ruthénium tels que le chlorure de ruthénium III, les composés de l'étain tels que le chlorure d'étain II, le chlorure d'étain IV, l'acétate d'étain II, l'octoate d'étain II, l'éthylhexanoate d'étain.

Un autre objet de l'invention concerne donc un catalyseur bimétallique comprenant de l'étain et du ruthénium, exempt de bore éventuellement sur un support caractérisé en ce qu'il comprend du ruthénium et de l'étain mis en oeuvre en quantités telles que le rapport molaire étain/ruthénium est d'au moins 2, de préférence compris entre 2 et 10 inclus et encore plus préférentiellement entre 2 et 6 inclus.

Dans les catalyseurs préférés, le rapport molaire est choisi entre 4 et 6.

La présente invention convient tout à fait bien à la préparation d'aldéhydes aromatiques et notamment les aldéhydes répndant à la formule :

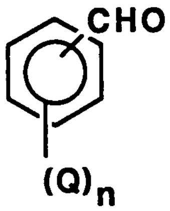

(V)

dans laquelle :

- n est égal à 0, 1, 2 ou 3,
- Q représente l'un des groupes ou fonctions suivantes :

. un atome d'hydrogène,
. un radical alkyle linéaire ou ramifié, ayant de 1 à 4 atomes de carbone,
. un radical alkoxy linéaire ou ramifié ayant de 1 à 4 atomes de carbone,
. un radical méthylène ou éthylène dioxy,
. un groupe -OH,
. un groupe -CHO,
. un groupe $NH_2$,
. un radical phényle,
. un atome d'halogène,
. un groupe $CF_3$.

L'invention permet la préparation de nombreu aldéhydes qui sont utilisés comme produits intermédiaires pharmaceutiques et/ou agrochimiques tels que par exemple, le difluoro-3,4 benzaldéhyde, le chloro-4 benzaldéhyde.

Il est particulièrement intéressant pour la préparation de l'aldéhyde salicylique qui peut être utilisé, entre autres, pour la préparation de la coumarine. L'aldéhyde salicylique obtenu selon le procédé de l'invention peut être engagé comme matière première dans la synthèse de la coumarine : celle-ci résultant d'une étape de cyclisation bien connue et largement décrite dans la littérature. On peut citer notamment la préparation de la coumarine selon la réaction de Perkin par réaction de l'aldéhyde salicylique et de l'anhydride acétique, en présence d'acétate de sodium (KIRK-OTHMER - Encyclopedia of Chemical Technology 7, p 198, 3 ème édition).

Le procédé de l'invention convient également pour la préparation d'autres aldéhydes aromatiques tels que l'hydroxy-3 benzaldéhyde, l'hydroxy-4 benzaldéhyde, la vanilline, le vératraldéhyde, le p-anisaldéhyde, le pipéronal.

La présente invention est également utilisable pour la synthèse d'aldéhydes divers. Elle peut servir pour préparer des aldéhydes saturés tels que le fluoral ou l'aldéhyde acétique. Elle est particulièrement adaptée à la synthèse d'aldéhydes insaturés, notamment dans la chimie des terpènes (prénal, citral ...), intermédiaires de synthèse de vitamines A ou E.

L'invention est particulièrement adaptée à la préparation d'aldéhydes de formule :

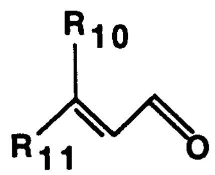

(VI)

dans laquelle $R_{10}$ et $R_{11}$, égaux ou différents, sont choisis parmi les radicaux alkyle ayant de 1 à 20 atomes de carbone environ, et les radicaux phényle et naphtyle éventuellement substitués par des radicaux alkyles ayant de 1 à 20 atomes de carbone.

A titre d'exemple d'aldéhydes, on peut citer le prénal et l'aldéhyde cinnamique.

Les exemples suivants, donnés à titre illustratif et non limitatif, permettent de compléter la présente description.

## EXEMPLE 1

Dans un tricol de 1 litre, on charge 0,37 g de $RuCl_3,xH_2O$ avec x égal à environ 2 (dissous auparavant dans 40 ml d'eau). On ajoute, sous agitation, en 30 minutes, 1,5 g de $SnCl_2,2H_2O$ dissous dans 120 ml d'eau.

Sous agitation, on ajoute alors 25 g d'alumine γ. On laisse agiter 15 minutes. On coupe alors l'agitation et on laisse reposer 16 heures. En fin de repos, on ajoute 25 ml d'eau, sous agitation.

On dissout 5,5 g de $NaBH_4$ dans 500 ml d'eau que l'on additionne goutte à goutte à température ambiante sur le catalyseur. La réduction se fait en deux temps :

- 1 heure sous agitation
- 16 heures au repos sans agitation.

Une fois la réduction effectuée, le catalyseur est filtré, puis lavé 5 fois avec 500 ml d'eau et 1 fois avec 500 ml d'éthanol.

Le catalyseur est alors séché à température ambiante. On obtient un catalyseur Ru-Sn-B/γ $Al_2O_3$ avec Sn/Ru = 4,7 (mol/mol) soit 1,2 % Ru (p/p), 3 % Sn (p/p).

EXEMPLE 2

Dans un réacteur tubulaire en verre de 18 mm de diamètre, on dispose 1 ml de catalyseur obtenu selon l'exemple 1 entre deux lits de 5 ml de quartz. On réduit le lit catalytique 1 heure à 450°C sous courant d'hydrogène 2,5 litres par heure. On abaisse ensuite la température à 240°C. Au moyen d'un pousse-seringue, on injecte alors la solution toluénique d'acide sénécioïque (18 % p/p acide/toluène) à un débit de 4 ml par heure. Le gaz issu de la réaction est condensé. Après deux heures de réaction, l'analyse du condensat donne le taux de transformation (TT) en acide sénécioïque et le rendement réel en prénal.

$$TT = \frac{\text{nombre de moles d'acide présentes au départ - nombre de moles d'acide restantes}}{\text{nombre de moles d'acide présentes au départ}}$$

$$RR = \frac{\text{nombre de moles obtenues}}{\text{nombre de moles engagées}}$$

$$RT = \frac{RR}{TT}$$

Au bout de 2 heures de réaction à 240°C, on obtient TT=65 %, RR=38 %.

EXEMPLE 3

Des catalyseurs à rapport variable Sn/Ru ont été préparés selon l'exemple 1 à teneur massique en ruthénium de 0,63 % (p/p). La réduction de l'acide sénécioïque en prénal a été effectuée selon l'exemple 2.

| Catalyseur RuSnB/$\gamma Al_2O_3$ Sn/Ru (mol/mol) | TT (%) Acide sénécioïque | RR(%) | RT(%) |
|---|---|---|---|
| | | Prénal | |
| 10 | 11 | 6,2 | 57 |
| 4,2 | 65 | 38 | 59 |
| 2 | 11 | 8,5 | 78,5 |
| 1 | 12 | 7,5 | 62 |

EXEMPLE 4

A rapport Sn/Ru fixe de 4,2 mol/mol, on prépare, selon l'exemple 1, des catalyseurs à teneur variable en ruthénium. La réduction de l'acide sénécioïque en prénal a été effectuée selon l'exemple 2.

| % Ru (p/p) | TT (%) Acide sénécioïque | RR (%) | RT (%) |
|---|---|---|---|
| | | Prénal | |
| 0,63 | 65 | 38 | 59 |
| 2 | 43 | 25,5 | 60 |
| 3,15 | 59,5 | 31,5 | 52,5 |

EXEMPLE 5

Différents supports ont été utilisés pour préparer des catalyseurs à O,63 % (p/p) Ru et Sn/Ru = 4,2 (mol/mol) suivant l'exemple 1. Ils sont comparés lors de la réduction de l'acide sénécioïque selon l'exemple décrit en 2.

| Support | TT (%) Acide sénécioïque | RR (%) | RT (%) |
|---|---|---|---|
| | | Prénal | |
| $\gamma Al_2O_3$ | 65 | 38 | 59 |
| $\alpha Al_2O_3$ | 41 | 34 | 83 |
| $SiO_2$ XOA 400 | 16 | 7,5 | 47,5 |
| $SiO_2$ XO 30LS | 30 | 27 | 89,5 |
| Noir d'acétylène | 26,5 | 25 | 96 |
| Noir CECA 3S | 26 | 12 | 44,5 |

EXEMPLE 6

La réduction de l'acide sénécioïque est comparée sur le catalyseur Ru-Sn-B/$\gamma Al_2O_3$ à 0,63 % (p/p) Ru et Sn/Ru = 4,2 activé 1 heure sous $H_2$, selon l'exemple 2, à différentes températures avant de faire la réaction à 240°C.

| Température (°C) d'activation | TT (%) Acide sénécioïque | RR (%) | RT (%) |
|---|---|---|---|
| | | Prénal | |
| 240 | 87 | 31 | 35 |
| 350 | 60 | 45 | 75 |
| 450 | 69 | 38 | 59 |

EXEMPLE 7

L'acide sénécioïque est dilué à 18 % (p/p) dans 3 solvants différents. On compare la réduction de l'acide selon l'exemple 2, sur un catalyseur à O,6 % Ru et Sn/Ru = 4,2 (mol/mol) sur $\gamma Al_2O_3$, réduit chimiquement avec $KBH_4$ selon l'exemple 1.

| Solvant | TT (%) Acide sénécioïque | RR (%) | RT (%) |
|---|---|---|---|
| | | Prénal | |
| toluène | 81,5 | 50 | 61 |
| DME | 80 | 56,5 | 70,5 |
| DME + $H_2O$ | 52 | 36 | 70 |

* $H_2O$ est ajouté en rapport stoechiométrique avec l'acide sénécioïque.

EXEMPLE 8

Les catalyseurs décrits peuvent être utilisés pour la synthèse de divers aldéhydes, dans les conditions de l'exemple 2.

| Acide à hydrogéner | TT (%) Acide | Aldéhyde recherché | RR (%) | RT (%) |
|---|---|---|---|---|
| 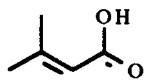 | 65 | 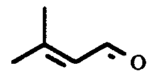 | 38 | 59 |
| 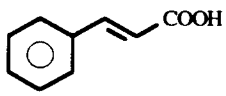 | | 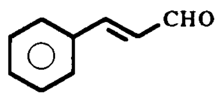 | Essai seulement qualitatif | |
| 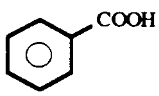 | 76 | 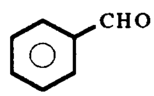 | 58 | 76 |
| 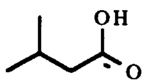 | 85,5 | 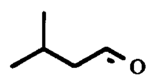 | 76 | 89 |

EXEMPLE 9

On opère selon l'exemple 2 en utilisant une solution d'acide dans le diméthoxyéthane. On obtient :

| Acide | T°C | TT | RR aldéhyde |
|---|---|---|---|
| 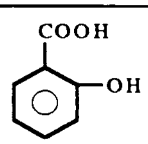 | 300°C | 90 | 10 % |
| 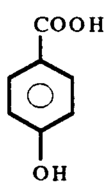 | 300°C | 85 | 50 % |

EXEMPLE 10

Dans un réacteur tubulaire en verre de 18 mm de diamètre, on dispose 2 ml de catalyseur obtenu selon l'exemple 1 en remplaçant la γ alumine par une $SiO_2$ XOL 30, entre deux lits de quartz de 5 ml. On réduit le lit catalytique 1 heure à 450°C sous courant d'hydrogène 2,5 litres par heure. On abaisse ensuite la température à 275°C. Au moyen d'un pousse seringue on injecte alors la solution toluénique d'acide 3,4-difluorobenzoïque 10 % p/v acide/toluène à un débit de 3 ml/h. Le gaz issu de la réaction est condensé. Après 2 heures de réaction, l'analyse du condensat donne le taux de transformation et le rendement réel en aldéhyde (RR).

TT = 100 %

RR = 65 %

A 300°C, en remplaçant $SiO_2$ XOL30 par $SiO_2$ OX50 (Degussa), on obtient les rendements suivants :

TT = 100 %
RR = 80 %.

EXEMPLE 11

Dans un réacteur tubulaire en verre de 18 mm de diamètre, on dispose 1 ml de catalyseur obtenu selon l'exemple 1 entre deux lits de quartz de 5 ml. On réduit le lit catalytique 1 heure à 450°C sous courant d'hydrogène 2 litres par heure. On abaisse ensuite la température à 300°C. Au moyen d'un pousse seringue on injecte alors la solution toluénique d'anhydride benzoïque 10 % p/v anhydride/toluène à un débit de 2 ml/h. Le gaz issu de la réaction est condensé. Après 6 heures de réaction, l'analyse du condensat donne le taux de transformation (TT) et le rendement réel en aldéhyde (RR).
TT = 86 %
RR = 39 %.

EXEMPLE 12

Dans un réacteur agité de 500 ml, on charge 0,375 g de $RuCl_3,xH_2O$ (dissous auparavant dans 40 ml d'eau). On ajoute, sous agitation, en 30 minutes, 1,5 g de $SnCl_2,2H_2O$ dissous dans 150 ml d'eau.

Sous agitation, on ajoute 25 g de $SiO_2$ 0X50 (Degussa). On laisse agiter 15 minutes. On coupe alors l'agitation et on laisse reposer 16 heures. En fin de repos, on évapore ensuite à sec en chauffant à 80°C sous 20 mm de mercure.

On sèche ensuite le résidu sous 20 mm de mercure à 40°C en étuve.

On obtient un catalyseur Ru-Sn/$SiO_2$ avec Sn/Ru = 4,0 (mol/mol) soit 0,6 % Ru (p/p).

EXEMPLE 13

Dans un réacteur tubulaire en verre de 18 mm de diamètre, on dispose 1 ml de catalyseur obtenu selon l'exemple 12 entre deux lits de 5 ml de quartz. On réduit le lit catalytique 1 heure à 450°C sous courant d'hydrogène 2,0 litres par heure. On abaisse ensuite la température à 200°C. Au moyen d'un pousse-seringue, on injecte alors de l'acide trifluoroacétique toujours sous courant d'hydrogène à 2,0 litres par heure à un débit de 1 ml par heure. Les gaz issus de la réaction sont condensés.

Après 5 heures de réaction, l'analyse du condensat donne les résultats suivants :

- $TT_{\text{acide trifluoroacétique}}$ = 68%
- $RR_{\text{fluoral monohydraté}}$ = 64 %.

EXEMPLE 14

Dans un réacteur tubulaire en verre de 18 mm de diamètre, on dispose 1 ml de catalyseur obtenu selon l'exemple 12 entre deux lits de 5 ml de quartz. On réduit le lit catalytique 1 heure à 450°C sous courant d'hydrogène 3,0 litres par heure. On abaisse ensuite la température à 400°C. Au moyen d'un pousse-seringue, on injecte alors de l'acide acétique toujours sous courant d'hydrogène à 3,0 litres par heure à un débit de 1 ml par heure. Les gaz issus de la réaction sont condensés.

Après 5 heures de réaction, l'analyse du condensat par chromatographie en phase gazeuse donne les résultats suivants :

- $TT_{\text{acide acétique}}$ = 87 %
- $RR_{\text{acétaldéhyde}}$ = 30 %.

EXEMPLE 15

Dans un réacteur tubulaire en verre de 18 mm de diamètre, on dispose 1 ml de catalyseur obtenu selon l'exemple 12 entre deux lits de 5 ml de quartz. On réduit le lit catalytique 1 heure à 450°C sous courant d'hydrogène 3,0 litres par heure. On abaisse ensuite la température à 300°C. Au moyen d'un pousse-seringue, on injecte alors une solution d'acide salicylique à 10 % en poids dans du diméthoxy-1,2 éthane toujours sous courant d'hydrogène à 3,0 litres par heure à un débit de 8 ml par heure. Les gaz issus de la réaction sont condensés.

Après 2 heures de réaction, l'analyse du condensat par chromatographie en phase gazeuse donne les résultats suivants :

- $TT_{\text{acide salicylique}}$ = 50 %
- $RR_{\text{aldéhyde salicylique}}$ = 30 %.

- $RR_{phénol}$ = 6 %.

EXEMPLE 16

Dans un ballon tricol équipé d'un thermomètre, d'une colonne à distiller, d'un rétrogradateur, d'un réfrigérant, d'un séparateur, on charge :

- de l'aldéhyde salicylique (600 mmol) préparé selon l'exemple précédent et récupéré par distillation,
- de l'anhydride acétique (1,90 mmol) en solution dans de l'acide acétique (3,47 g).

On porte à reflux et l'on introduit de l'acétate de sodium (2,1 mmol) en solution dans de l'acide acétique (3,47 g).

On distille l'acide acétique en maintenant un reflux tel que la température de tête de colonne soit voisine de 118°C.

Après 2 heures 50 de réaction, on dose par chromatographie en phase gazeuse, la coumarine dans le bouilleur ce qui permet de déterminer un rendement en coumarine de 81 %.

## Revendications

1. Procédé de préparation d'aldéhydes et de leurs dérivés par réduction à l'hydrogène d'acides, d'esters ou d'anhydrides carboxyliques caractérisé en ce que l'on opère en phase vapeur, en présence d'un catalyseur bimétallique de type ruthénium/étain.

2. Procédé selon la revendication 1 caractérisé en ce que le catalyseur est un catalyseur ruthénium-étain-bore.

3. Procédé selon la revendication 2 caractérisé en ce que la teneur en bore est inférieure à 1 % et de préférence voisine de 0,5 %.

4. Procédé selon la revendication 1 caractérisé en ce que le catalyseur est un catalyseur bimétallique ruthénium-étain exempt de bore.

5. Procédé selon la revendication 1 caractérisé en ce que le catalyseur est tel que le rapport molaire entre l'étain et le ruthénium engagés Sn/Ru est compris entre 1 et 10 inclus, et de préférence entre 2 et 6 inclus.

6. Procédé selon l'une quelconque des revendications 1 à 5 caractérisé en ce que le ruthénium représente entre 0,1 % et 50 % environ du poids du catalyseur.

7. Procédé selon la revendication 6 caractérisé en ce que le ruthénium représente de 10 à 50 % du poids du catalyseur lorsqu'il est massique et de 0,1 à 20 %, de préférence de 0,5 à 3 % du poids du catalyseur lorsqu'il est supporté.

8. Procédé selon l'une quelconque des revendications 1 à 7 caractérisé en ce que l'on utilise un catalyseur supporté.

9. Procédé selon la revendication 8 caractérisé en ce que le support est choisi parmi les oxydes de métaux, les charbons et les résines.

10. Procédé selon la revendication 9 caractérisé en ce que le support est un oxyde d'aluminium et/ou de silicium.

11. Procédé selon l'une des revendications 8 à 10 caractérisé en ce que le support a une surface spécifique (B.E.T.) choisie entre 50 et 100 $m^2/g$ lorsque la teneur en ruthénium du catalyseur est comprise entre 0,1 et 1 %.

12. Procédé selon la revendication 1 caractérisé en ce que l'on opère à une température comprise entre 150°C et 500°C, et de préférence, entre 200 et 400°C.

13. Procédé selon l'une des revendications 1 à 12 caractérisé en ce que l'on effectue une activation préalable du catalyseur, par forte élévation de température, de préférence à des températures proches de 500°C environ, et préférentiellement de 450°C ; l'activation étant conduite de préférence sous courant d'hydrogène.

14. Procédé selon la revendication 1 caractérisé en ce que pour 1 ml de catalyseur, l'hydrogène est injecté à un débit compris entre 0,1 et 10 litres par heure, et l'acide, l'ester ou l'anhydride, à un débit liquide au plus égal à 10 ml/h, et de préférence compris entre 0,5 et 5 ml/h.

**15.** Procédé selon la revendication 1 caractérisé en ce que l'acide carboxylique, l'ester ou l'anhydride est injecté directement sous forme gazeuse.

**16.** Procédé selon la revendication 1 caractérisé en ce que l'acide carboxylique, l'ester ou l'anhydride est injecté sous forme liquide dans un solvant inerte.

**17.** Procédé selon la revendication 1 caractérisé en ce que l'hydrogène est injecté à la pression atmosphérique ou sous une légère pression, éventuellement dilué dans un gaz inerte.

**18.** Procédé selon l'une des revendications 1 à 17 pour la préparation d'aldéhydes de formule générale :

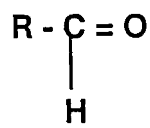

(I)

dans laquelle R représente un atome d'hydrogène ou un radical hydrocarboné, éventuellement substitué comportant de 1 à 40 atomes de carbone qui peut être un radical aliphatique acyclique saturé ou insaturé, linéaire ou ramifié ; un radical carbocyclique ou hétérocyclique saturé, insaturé ou aromatique, monocyclique ou polycyclique, par réduction d'esters, d'anhydrides ou d'acides de formule :

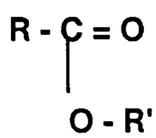

(II)

dans laquelle :

- R est défini comme précédemment,
- R' représente :
  - un groupement R tel que précédemment défini,
  - un groupement

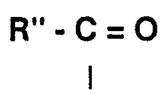

  dans lequel R'' a la signification donnée pour R,
  - les deux groupements R et R'' pouvant être liés entre eux pour former un cycle saturé ou insaturé ayant de 5 à 7 atomes et comprenant la fonction anhydride,
  - les deux groupements R et R'' par l'intermédiaire de deux atomes vicinaux pouvant former ensemble un pont d'un système bicyclique orthocondensé.

**19.** Procédé selon la revendication 18 caractérisé en ce que l'acide carboxylique ou dérivé répond à la formule (II) dans laquelle le groupement R représente un radical hydrocarboné éventuellement substitué comportant de 1 à 20 atomes de carbone.

**20.** Procédé selon l'une des revendications 18 et 19 caractérisé en ce que l'acide carboxylique ou dérivé répond à la formule (II) dans laquelle le groupement R représente préférentiellement un reste hydrocarboné aromatique, et

notamment benzénique répondant à la formule générale (III) :

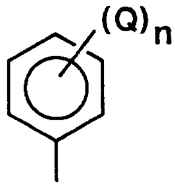

(III)

dans ladite formule (III) :

- n est un nombre entier de 0 à 5, de préférence de 0 à 3,
- Q représente $R_1$, l'un des groupes ou fonctions suivantes :

  . un radical alkyle, linéaire ou ramifié, ayant de 1 à 6 atomes de carbone, de préférence de 1 à 4 atomes de carbone, tel que méthyle, éthyle, propyle, isopropyle, butyle, isobutyle, sec-butyle, tert-butyle,
  . un radical alcényle linéaire ou ramifié ayant de 2 à 6 atomes de carbone, de préférence, de 2 à 4 atomes de carbone, tel que vinyle, allyle,
  . un radical alkoxy linéaire ou ramifié ayant de 1 à 6 atomes de carbone, de préférence de 1 à 4 atomes de carbone tel que les radicaux méthoxy, éthoxy, propoxy, isopropoxy, butoxy,
  . un groupe acyle ayant de 2 à 6 atomes de carbone,
  . un radical de formule :

$$-R_2-OH$$

$$-R_2-COOR_5$$

$$-R_2-CHO$$

$$-R_2-NO_2$$

$$-R_2-CN$$

$$-R_2-(NR_5)_2$$

$$-R_2-CO-(NR_5)_2$$

$$-R_2-SH$$

$$-R_2-X$$

$$-R_2-CF_3$$

dans lesdites formules, $R_2$ représente un lien valentiel ou un radical hydrocarboné divalent, linéaire ou ramifié, saturé ou insaturé, ayant de 1 à 6 atomes de carbone tel que, par exemple, méthylène, éthylène, propylène, isopropylène, isopropylidène ; $R_5$ représente un atome d'hydrogène ou un radical alkyle linéaire ou ramifié ayant de 1 à 6 atomes de carbone; X symbolise un atome d'halogène, de préférence un atome de chlore, de brome ou de fluor.

- Q représente $R_3$ l'un des radicaux plus complexes suivants :

  . un radical

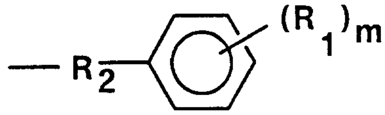

  dans lequel $R_1$ et $R_2$ ont la signification donnée précédemment et m est un nombre entier de 0 à 5, de préférence de 0 à 3,

  . un radical $-R_2-A-R_4$ dans lequel $R_2$ a la signification donnée précédemment, $R_4$ représente un radical alkyle linéaire ou ramifié ayant de 1 à 6 atomes de carbone, de préférence, de 1 à 4 atomes de carbone ou un radical de formule

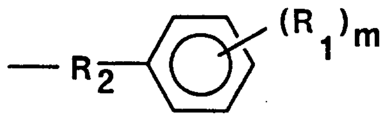

  et A symbolise l'un des groupes suivants :
  -O-, -CO-,

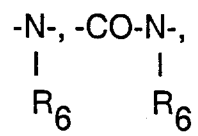

  -S-, $-SO_2-$
  dans ces formules, $R_6$ représente un atome d'hydrogène ou un radical alkyle linéaire ou ramifié ayant de 1 à 4 atomes de carbone, de préférence, un radical méthyle ou éthyle.

**21.** Procédé selon la revendication 20 caractérisé en ce que l'acide carboxylique ou dérivé répond à la formule (II) dans laquelle le groupement R représente un reste hydrocarboné aromatique répondant à la formule générale (III) dans laquelle lorsque n est supérieur à 1, les radicaux Q peuvent être identiques ou différents et 2 atomes de carbone successifs du cycle benzénique peuvent être reliés entre eux par un pont cétalique tels que les radicaux méthylène dioxy ou éthylène dioxy extranucléaires.

**22.** Procédé selon la revendication 20 caractérisé en ce que l'acide carboxylique ou dérivé répond à la formule générale (II) dans laquelle le groupement R représente un reste aromatique répondant à la formule générale (III) dans laquelle :

- n est égal à 0, 1, 2 ou 3,
- Q représente l'un des groupes ou fonctions suivantes :

  . un atome d'hydrogène,
  . un radical alkyle linéaire ou ramifié, ayant de 1 à 4 atomes de carbone,
  . un radical alkoxy linéaire ou ramifié ayant de 1 à 4 atomes de carbone,
  . un radical méthylène ou éthylène dioxy,
  . un groupe -OH,
  . un groupe -CHO,
  . un groupe $NH_2$,
  . un radical phényle,
  . un atome d'halogène,
  . un groupe $CF_3$.

**23.** Procédé selon l'une des revendications 18 et 19 caractérisé en ce que l'acide carboxylique ou dérivé répond à la formule générale (II) dans laquelle le groupement R représente un reste naphtalénique : lesdits cycles pouvant être substitués par 1 à 4 radicaux $R_1$ de préférence de 1 à 3, $R_1$ ayant les significations énoncées précédemment dans la revendication 20.

**24.** Procédé selon l'une des revendications 18 et 19 caractérisé en ce que l'acide carboxylique ou dérivé répond à la formule générale (II) dans laquelle le groupement R représente un reste carbocyclique saturé ou comprenant 1 ou 2 insaturations dans le cycle, ayant généralement de 3 à 7 atomes de carbone, de préférence, 6 atomes de carbone dans le cycle ; ledit cycle pouvant être substitué par 1 à 5 radicaux $R_1$, de préférence 1 à 3, $R_1$ ayant les significations énoncées précédemment dans la revendication 20.

**25.** Procédé selon l'une des revendications 18 et 19 caractérisé en ce que l'acide carboxylique ou dérivé répond à la formule générale (II) dans laquelle le groupement R représente un reste aliphatique acyclique linéaire ou ramifié ayant de préférence de 1 à 12 atomes de carbone, saturé ou comprenant une à plusieurs insaturations sur la chaîne, généralement, 1 à 3 insaturations qui peuvent être des doubles liaisons simples ou conjuguées ou des triples liaisons ; la chaîne hydrocarbonée peut être éventuellement :

- interrompue par l'un des groupes suivants :
  -O-, -CO-,

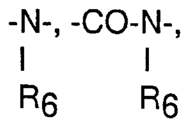

  -S-, $-SO_2-$
  dans ces formules $R_6$ représente l'hydrogène ou un radical alkyle linéaire ou ramifié ayant de 1 à 4 atomes de carbone, de préférence, un radical méthyle ou éthyle,
- et/ou porteuse de l'un des substituants suivants :
  -OH, $-COOR_5$, -CHO, $-NO_2$, -CN, $-NH_2$, -SH, -X, $-CF_3$,
  dans ces formules, $R_5$ ayant la signification donnée précédemment dans la revendication 20.

**26.** Procédé selon l'une des revendications 18 et 19 caractérisé en ce que l'acide carboxylique ou dérivé répond à la formule générale (II) dans laquelle le groupement R répond à la formule suivante :

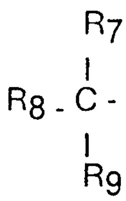
(IV)

dans laquelle $R_7$, $R_8$ et $R_9$, identiques ou différents, sont choisis parmi un atome d'hydrogène, un radical alkyle linéaire ou ramifié contenant 1 à 10 atomes de carbone, un radical alcényle linéaire ou ramifié contenant 1 à 10 atomes de carbone, un radical alkoxy linéaire ou ramifié contenant 1 à 10 atomes de carbone, un groupe hydroxyle, une fonction amine ou un atome d'halogène ou un groupe $-CF_3$.

**27.** Procédé selon la revendication 26 caractérisé en ce que l'acide carboxylique ou dérivé répond à la formule générale (II) dans laquelle le groupement R répond à la formule (IV) dans laquelle l'un des 3 groupements $R_7$, $R_8$ et $R_9$ possède un atome de carbone formant une double liaison conjuguée avec le groupement carbonyle de l'acide, de l'ester ou de l'anhydride carboxylique.

**28.** Procédé selon l'une des revendications 18 et 19 caractérisé en ce que l'acide carboxylique ou dérivé répond à la formule générale (II) dans laquelle le groupement R représente un reste aliphatique acyclique, saturé ou insaturé, linéaire ou ramifié qui peut être éventuellement porteur d'un substituant cyclique, ledit reste aliphatique acyclique pouvant être relié au cycle par un lien valentiel ou par l'un des groupes suivants :

-O-, -CO-,

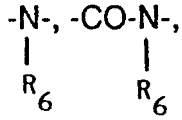

-S-, $-SO_2-$

dans ces formules, $R_6$ ayant la signification donnée précédemment dans la revendication 20.

**29.** Procédé selon l'une des revendications 18 et 19 caractérisé en ce que l'acide carboxylique ou dérivé répond à la formule générale (II) dans laquelle le groupement R représente un reste hétérocyclique, saturé ou non, comportant notamment 5 ou 6 atomes dans le cycle dont 1 ou 2 hétéroatomes tels que les atomes d'azote, de soufre et d'oxygène ; les atomes de carbone de l'hétérocycle pouvant éventuellement être substitués, dans leur totalité ou pour une partie d'entre eux seulement par des radicaux $R_1$, $R_1$ ayant les significations énoncées précédemment dans la revendication 20 ou le groupement R représente un reste hétérocyclique polycyclique.

**30.** Procédé selon l'une des revendications 1 à 29 caractérisé en ce que le substrat de départ est tout acide carboxylique mono- ou polycarboxylique tels que les acides aliphatiques saturés ou insaturés ; carbocycliques ou hétérocycliques, saturés, insaturés ou aromatiques, monocycliques ou polycycliques ; aliphatiques saturés ou insaturés porteurs d'un substituant cyclique tel qu'un cycle carbocyclique ou hétérocyclique saturé, insaturé ou aromatique.

**31.** Procédé selon l'une des revendications 1 à 30 caractérisé en ce que l'acide carboxylique ou dérivé répondant a la formule générale (II) est choisi parmi :

- les acides monocarboxyliques aliphatiques saturés
- les acides dicarboxyliques aliphatiques saturés
- les acides monocarboxyliques ou dicarboxyliques aliphatiques insaturés
- les acides carboxyliques carbocycliques saturés ou insaturés
- les acides carboxyliques hétérocycliques
- les acides carboxyliques carbocycliques aromatiques
- les acides carboxyliques arylaliphatiques saturés ou insaturés
- les acides carboxyliques aliphatiques ou aromatiques halogénés
- les hydroxy-acides aliphatiques, cycloaliphatiques, arylaliphatiques
- les acides hydroxybenzoïques
- les alcoxy- et phénoxyacides
- les oxo-acides
- les acyloxy-acides
- les amido-acides
- les acides aminés éventuellement N- protégés.

**32.** Procédé selon l'une des revendications 1 à 31 caractérisé en ce que l'acide carboxylique ou dérivé répondant à la formule générale (II) est choisi parmi :

- les acides monocarboxyliques aliphatiques saturés tels que l'acide formique, acétique, propionique, butyrique, isobutyrique, valérique, isovalérique, pivalique, laurique, myristique, palmitique, stéarique,
- les acides dicarboxyliques aliphatiques saturés tels que l'acide oxalique, malonique, succinique, glutarique, adipique, pimélique, subérique, azélaïque, sébacique,
- les acides monocarboxyliques ou dicarboxyliques aliphatiques insaturés tels que l'acide acrylique, propiolique, méthacrylique, crotonique, isocrotonique, sénécioïque, acide tiglique, oléïque, maléique, fumarique, citraconique, mésaconique,
- les acides carboxyliques carbocycliques saturés ou insaturés tels que l'acide camphorique, l'acide chrysanthémique,
- les acides carboxyliques hétérocycliques tels que les acides furannecarboxyliques, thiophènecarboxyliques, pyrrolecarboxyliques, pyrazinecarboxyliques, l'acide nicotinique, isonicotinique, l'acide picolinique,
- les acides carboxyliques carbocycliques aromatiques tels que l'acide benzoïque, phtalique, isophtalique, téréphtalique, les acides naphtalènecarboxyliques, les acides toluiques,

- les acides carboxyliques arylaliphatiques saturés tels que, notamment les arylpropioniques comme l'acide phényl-2 propionique, l'acide [(butyl-2)-4 phényl]-2 propionique, l'acide (benzoyl-3 phényl)-2 propionique, l'acide (méthoxy-6 naphtyl-2)-2 propionique ou les acides insaturés comme par exemple l'acide phényl-2 propénoïque, l'acide cinnamique,
- les acides carboxyliques aliphatiques ou aromatiques halogénés tels que l'acide monofluoroacétique, difluroacétique, monochloroacétique, dichloroacétique, trichloroacétique, monochloropropionique, $\alpha$-bromopropionique, $\alpha$-bromobutyrique, trifluoroacétique, l'acide monofluoro-o-benzoïque, l'acide monofluoro-m-benzoïque, l'acide monofluoro-p-benzoïque, l'acide difluoro-2,3 benzoïque, l'acide difluoro-2,4 benzoïque, l'acide difluoro-2,5 benzoïque, l'acide difluoro-3,4 benzoïque, l'acide trifluoro-2,3,6 benzoïque, l'acide trifluoro-2,4,5 benzoïque, l'acide tétrafluoro-2,3,4,5 benzoïque, l'acide pentafluorobenzoïque, l'acide $\alpha,\alpha,\alpha$-trifluoro-o-toluique, l'acide $\alpha,\alpha,\alpha$-trifluoro-m-toluique, l'acide $\alpha,\alpha,\alpha$-trifluoro-p-toluique, l'acide monochloro-o-benzoïque, l'acide monochloro-m-benzoïque, l'acide monochloro-p-benzoïque, l'acide dichloro-2,3 benzoïque, l'acide dichloro-2,4 benzoïque, l'acide dichloro-2,5 benzoïque, l'acide dichloro-2,6 benzoïque, l'acide dichloro-3,4 benzoïque, l'acide dichloro-3,5 benzoïque, l'acide trichloro-2,3,5 benzoïque, l'acide trichloro-2,3,6 benzoïque, l'acide chloro-2 fluoro-4,5 benzoïque, l'acide chloro-3 trifluro-2,4,5 benzoïque, l'acide monobromo-o-benzoïque, l'acide monobromo-m-benzoïque, l'acide monobromo-p-benzoïque.
- les hydroxy-acides aliphatiques, cycloaliphatiques, arylaliphatiques, tels que l'acide glycolique, l'acide lactique, l'acide glycérique, l'acide hydroxy-2 butanoïque, l'acide hydroxy-3 butanoïque, l'acide méthyl-2 lactique, l'acide hydroxy-2 méthylthio-4 butanoïque, l'acide tartronique, l'acide malique, l'acide tartrique, l'acide hydroxy-1 cyclopropane carboxylique, l'acide hydroxy-2 phénylpropanoïque, l'acide hydroxy-2 cinnamique, l'acide hydroxy-3 cinnamique, l'acide hydroxy-4 cinnamique,
- les acides hydroxybenzoïques suivants : l'acide hydroxy-2 benzoïque (acide salicylique), l'acide hydroxy-3 benzoïque, l'acide hydroxy-4 benzoïque, l'acide méthyl-3 salicylique, l' acide méthyl-4 salicylique, l'acide méthyl-5 salicylique, l'acide hydroxy-3 méthyl-4 benzoïque, l'acide méthoxy-3 salicylique, l'acide méthoxy-4 salicylique, l'acide méthoxy-5 salicylique, l'acide hydroxy-3 méthoxy-4 benzoïque (acide isovanillique), l'acide hydroxy-4 méthoxy-3 benzoïque (acide vanillique), l'acide hydroxy-3 diméthoxy-4,5 benzoïque, l'acide hydroxy-4 diméthoxy-3,5 benzoïque (acide syringique), l'acide hydroxy-5 isophtalique, l'acide amino-3 salicylique, l'acide amino-4 salicylique, l'acide amino-5 salicylique, l'acide hydroxy-3 amino-2 benzoïque, l'acide nitro-3 salicylique, l'acide hydroxy-3 nitro-4 benzoïque, l'acide hydroxy-4 nitro-3 benzoïque, l'acide hydroxy-3 méthyl-4 nitro-2 benzoïque, l'acide diiodo-3,5 salicylique, l'acide dihydroxy-2,3 benzoïque, l'acide dihydroxy-2,4 benzoïque, l'acide dihydroxy-2,5 benzoïque, l'acide dihydroxy-2,6 benzoïque, l'acide dihydroxy-3,4 benzoïque (acide protocatéchique), l'acide dihydroxy-3,5 benzoïque, l'acide dihydroxy-3,5 méthyl-4 benzoïque, l'acide trihydroxy-2,3,4 benzoïque, l'acide trihydroxy-2,4,6 benzoïque, l'acide trihydroxy-3,4,5 benzoïque,
- les alcoxy- et phénoxyacides tels que l'acide méthoxyacétique, phénoxyacétique, dichloro-2,4 phénoxyacétique, phénoxypropionique, dichloro-2,4 phénoxypropionique, p-hydroxyphénoxypropionique, m-chlorophénoxypropionique, l'acide phénoxy-4 benzoïque, l'acide (carboxy-4 phénoxy-4) benzoïque, l'acide pipéronylique,
- les oxo-acides tels que l'acide acétyl-2 benzoïque, l'acide acétyl-4 benzoïque, l'acide benzoyl-2 benzoïque, l'acide benzoyl-4 benzoïque,
- les acyloxy-acides tels que l'acide benzoyloxy-3 propionique, l'acide acétoxy-2 benzoïque, l'acide acétoxy-4 benzoïque,
- les amido-acides tels que l'acide acétamido-2 acrylique, l'acide acétamido-2 benzoïque, l'acide acétamido-3 benzoïque, l'acide N-acétamido-4 benzoïque,
- les acides aminés éventuellement N- protégés par un groupe protecteur comme par exemple les groupes suivants acyle (acétyle, benzoyle), BOC (butyloxycarbonyl), CBZ (carbobenzoxy), FMOC (fluorényl-9 méthoxycarbonyl), MSOC (méthanesulfényl-2 éthoxycarbonyl).

**33.** Procédé selon la revendication 32 caractérisé en ce que l'acide carboxylique ou dérivé répondant à la formule générale (II) est choisi parmi :

- les acides carboxyliques ou dicarboxyliques saturés ou insaturés,
- l'acide salicylique et l'acide hydroxy-4 benzoïque.
- l'acide acétique, l'acide propionique et leurs dérivés substitués par un groupe hydroxy, halogène, phényle, phényloxy,
- l'acide benzoïque et ses dérivés substitués par un groupe alkyle $C_1$ -$C_4$, acétoxy, acétamido, hydroxy, méthoxy, éthoxy,
- les acides carboxyliques aliphatiques ou aromatiques halogénés,
- l'acide nicotinique.

**34.** Procédé selon l'une des revendications 1 à 33 caractérisé en ce que l'aldéhyde préparé répond plus particulièrement à la formule générale (V) :

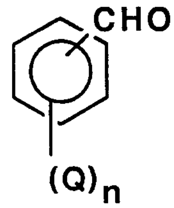

(V)

dans laquelle :

- n est égal à 0, 1, 2 ou 3,
- Q représente l'un des groupes ou fonctions suivantes :

  . un atome d'hydrogène,
  . un radical alkyle linéaire ou ramifié, ayant de 1 à 4 atomes de carbone,
  . un radical alkoxy linéaire ou ramifié ayant de 1 à 4 atomes de carbone,
  . un radical méthylène ou éthylène dioxy,
  . un groupe -OH,
  . un groupe -CHO,
  . un groupe $NH_2$,
  . un radical phényle,
  . un atome d'halogène,
  . un groupe $CF_3$.

**35.** Procédé selon l'une des revendications 1 à 34 caractérisé en ce que l'aldéhyde préparé est plus particulièrement le difluoro-3,4 benzaldéhyde, le chloro-4 benzaldéhyde, l'aldéhyde salicylique, l'hydroxy-3 benzaldéhyde, l'hydroxy-4 benzaldéhyde, la vanilline, le vératraldéhyde, le p-anisaldéhyde, l'aldéhyde cinnamique, le pipéronal, le fluoral, l'aldéhyde acétique, le prénal, le citral.

**36.** Utilisation de l'aldéhyde salicylique obtenu selon le procédé de préparation décrit dans l'une des revendications 1 à 33 comme intermédiaire de fabrication de la coumarine.

**37.** Catalyseur bimétallique comprenant de l'étain et du ruthénium, exempt de bore éventuellement sur un support caractérisé en ce qu'il comprend du ruthénium et de l'étain mis en oeuvre en quantités telles que le rapport molaire étain/ruthénium est d'au moins 2, de préférence compris entre 2 et 10 inclus et encore plus préférentiellement entre 2 et 6 inclus.

**38.** Catalyseur selon la revendication 37 caractérisé en ce qu'il est préparé par dépôt, d'une manière connue, des éléments métalliques sur le support puis en soumettant la masse de contact à une réduction au moyen de l'hydrogène.

**39.** Catalyseur selon l'une quelconque des revendications 37 et 38 caractérisé en ce que le ruthénium représente entre 0,1 % et 50 % environ du poids du catalyseur.

**40.** Catalyseur selon la revendication 39 caractérisé en ce que le ruthénium représente de 10 à 50 % du poids du catalyseur lorsqu'il est massique et de 0,1 à 20 %, de préférence de 0,5 à 3 % du poids du catalyseur lorsqu'il est supporté.

**41.** Catalyseur selon la revendication 37 caractérisé en ce qu'il est supporté et en ce que le support est choisi parmi les oxydes de métaux, de préférence, les oxydes d'aluminium et/ou de silicium, les charbons, le noir d'acétylène et les résines.

**42.** Catalyseur selon l'une des revendications 37 à 41 caractérisé en ce que le ruthénium est introduit sous la forme de ruthénium métallique, de chlorure de ruthénium III, de chlorure de ruthénium IV, de pentafluorure de ruthénium,

d'oxyde de ruthénium II, d'oxyde de ruthénium IV, d'oxychlorure de ruthénium ammoniaqué, d'acétate de ruthénium, de préférence de chlorure de ruthénium III.

43. Catalyseur selon l'une des revendications 37 à 42 caractérisé en ce que l'étain est introduit sous la forme d'étain métallique, d'oxydes, de chlorures, de nitrates, de carboxylates, d'alcoolates d'étain ou de composés organométalliques dans lesquels l'étain est lié à un atome d'hydrogène et/ou des radicaux alkyle ayant de préférence de 1 à 4 atomes de carbone, de préférence de chlorure d'étain II, de chlorure d'étain IV, d'acétate d'étain II, d'octoate d'étain II, d'éthylhexanoate d'étain.

## Claims

1. A process for the preparation of aldehydes and their derivatives by hydrogen reduction of carboxylic acids, esters or anhydrides, characterised in that the operation is carried out in vapour phase, in the presence of a bimetallic catalyst of the ruthenium/tin type.

2. A process according to Claim 1, characterised in that the catalyst is a ruthenium-tin-boron catalyst.

3. A process according to Claim 2, characterised in that the boron content is less than 1% and preferably close to 0.5%.

4. A process according to Claim 1, characterised in that the catalyst is a bimetallic ruthenium-tin catalyst free from boron.

5. A process according to Claim 1, characterised in that the catalyst is such that the Sn/Ru tin/ruthenium molar ratio used is between 1 and 10 inclusive, preferably between 2 and 6 inclusive.

6. A process according to any one of Claims 1 to 5, characterised in that the ruthenium represents between 0.1% and 50% approximately by weight of the catalyst.

7. A process according to Claim 6, characterised in that the ruthenium represents from 10 to 50% by weight of the catalyst when in mass, and from 0.1 to 20%, preferably from 0.5 to 3% by weight of the catalyst when it is supported.

8. A process according to any one of Claims 1 to 7, characterised in that a supported catalyst is used.

9. A process according to Claim 8, characterised in that the support is selected from metal oxides, carbons and resins.

10. A process according to Claim 9, characterised in that the support is an aluminium oxide and/or silicon.

11. A process according to one of Claims 8 to 10, characterised in that the support has a specific surface (B.E.T.) which is selected between 50 and 100 $m^2/g$ when the ruthenium content of the catalyst is between 0.1 and 1%.

12. A process according to Claim 1, characterised in that the operation is carried out at a temperature of between 150°C and 500°C, preferably of between 200 and 400°C.

13. A process according to one of Claims 1 to 12, characterised in that the catalyst is activated beforehand, by greatly increasing the temperature, preferably to temperatures close to about 500°C, and preferably close to 450°C: the activation preferably being carried out in a flow of hydrogen.

14. A process according to Claim 1, characterised in that for 1 ml of catalyst, the hydrogen is injected at a flow rate of between 0.1 and 10 l/h, and the acid, ester or anhydride at a flow rate at the most equal to 10 ml/h, and preferably between 0.5 and 5 ml/h.

15. A process according to Claim 1, characterised in that the carboxylic acid, ester or anhydride is injected directly in gaseous form.

16. A process according to Claim 1, characterised in that the carboxylic acid, ester or anhydride is injected in liquid form into an inert solvent.

17. A process according to Claim 1, characterised in that the hydrogen is injected at atmospheric pressure or at low pressure, possibly diluted in an inert gas.

**18.** A process according to one of Claims 1 to 17 for the preparation of aldehydes of the general formula:

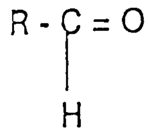

(I)

wherein R represents a hydrogen atom or a hydrocarbon radical, possibly substituted, having 1 to 40 carbon atoms and which can be a saturated or unsaturated, straight or branched, acyclic aliphatic radical; a saturated, unsaturated, or aromatic, monocyclic or polycyclic, carbocyclic or heterocyclic radical by the reduction of esters, anhydrides or acids of formula:

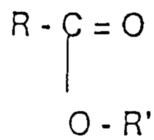

(II)

wherein:

- R is defined as hereinabove,
- R' represents:
  - an R grouping as defined hereinabove,
  - an

    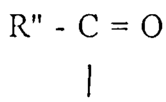

    grouping in which R" has the meaning given for R
  - the two R and R" groupings being able to be linked together to form a saturated or unsaturated cycle having 5 to 7 atoms and comprising the anhydride function,
  - the two R and R" groupings by the intermediary of two neighbouring atoms being able to form together a bridge of an orthocondensed bicyclic system.

**19.** A process according to Claim 18, characterised in that the carboxylic acid or derivative corresponds to Formula (II) in which the group R represents a hydrocarbon radical, possibly substituted, comprising 1 to 20 carbon atoms.

**20.** A process according to one of Claims 18 and 19, characterised in that the carboxylic acid or derivative corresponds to Formula (II) in which the grouping R preferably represents an aromatic hydrocarbon residue, in particular benzene, corresponding to general formula (III):

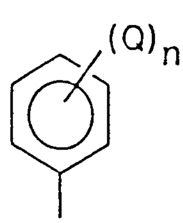

(III)

in which formula (III):

- n is a whole number from 0 to 5, preferably from 0 to 3,
- Q represents $R_1$, one of the following groups or functions:

  . an straight or branched alkyl radical with 1 to 6 carbon atoms, preferably 1 to 4 carbon atoms, such as methyl, ethyl, propyl, isopropyl, butyl, isobutyl, sec-butyl, tert-butyl,
  . a straight or branched alkenyl radical with 2 to 6 carbon atoms, preferably with 2 to 4 carbon atoms, such as vinyl, allyl,
  . a straight or branched alkoxy radical with 1 to 6 carbon atoms, preferably 1 to 4 carbon atoms, such as methoxy, ethoxy, propoxy, isopropoxy, butoxy radicals,
  . an akyl group with 2 to 6 carbon atoms,
  . a radical of formula:

$$-R_2-OH$$

$$-R_2-COOR_5$$

$$-R_2-CHO$$

$$-R_2-NO_2$$

$$-R_2-CN$$

$$-R_2-(NR_5)_2$$

$$-R_2-CO-(NR_5)_2$$

$$-R_2-SH$$

$$-R_2-X$$

$$-R_2-CF_3$$

  in which formulae, $R_2$ represents a valency bond or a saturated or unsaturated, straight or branched, divalent hydrocarbon radical with 1 to 6 carbon atoms, such as methylene, ethylene, propylene, isopropylene, isopropylidene, for example; $R_5$ represents a hydrogen atom or a straight or branched alkyl radical with 1 to 6 carbon atoms; X symbolises a halogen atom preferably a chlorine, bromine or fluorine atom.

- Q represents $R_3$, one of the following more complex radicals:

  . a radical

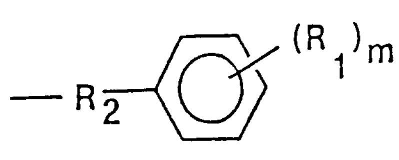

  wherein $R_1$ and $R_2$ have the meaning given hereinabove, and m is a whole number from 0 to 5, preferably from 0 to 3,

. a $-R_2-A-R_4$ radical wherein $R_2$ has the meaning given hereinabove, $R_4$ represents a straight or branched, alkyl radical with 1 to 6 carbon atoms, preferably 1 to 4 carbon atoms, or a radical of the formula

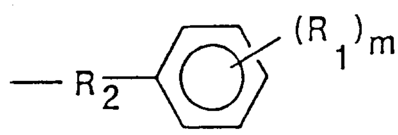

and A symbolises one of the following groups:
-O-, -CO-,

-N-, -CO-N-,
| |
$R_6$ $R_6$

-S-, $-SO_2-$
in which formulae, $R_6$ represents a hydrogen atom or a straight or branched alkyl radical with 1 to 4 carbon atoms, preferably a methyl or ethyl radical.

**21.** A process according to Claim 20, characterised in that the carboxylic acid or derivative corresponds to formula (II) wherein the R grouping represents an aromatic hydrocarbon residue corresponding to general formula (III), wherein, when n is greater than 1, the Q radicals can be the same or different, and 2 successive carbon atoms of the benzene cycle can be interconnected by a ketal bridge such as extranuclear methylene dioxy or ethylene dioxy radicals.

**22.** A process according to Claim 20, characterised in that the carboxylic acid or derivative corresponds to general formula (II) wherein the R grouping represents an aromatic residue corresponding to general formula (III) in which:

- n is equal to 0, 1, 2 or 3,
- Q represents one of the following groups or functions:

  . a hydrogen atom
  . a straight or branched alkyl radical, with 1 to 4 carbon atoms,
  . a straight or branched alkoxy radical with 1 to 4 carbon atoms,
  . a methylene or ethylene dioxy radical,
  . an -OH group,
  . a -CHO group,
  . a $NH_2$ group,
  . a phenyl radical,
  . a halogen atom,
  . a $CF_3$ group

**23.** A process according to one of Claims 18 and 19, characterised in that the carboxylic acid or derivative corresponds to general formula (II) wherein the R grouping represents a naphthalene residue: said cycles can be substituted by 1 to 4 radicals $R_1$, preferably by 1 to 3, $R_1$ having the meanings mentioned hereinabove in Claim 20.

**24.** A process according to one of Claims 18 and 19, characterised in that the carboxylic acid or derivative corresponds to general formula (II), in which the R grouping represents a saturated carbocyclic residue or comprising 1 to 2 insaturations in the cycle, usually with 3 to 7 carbon atoms, preferably with 6 carbon atoms, in the cycle; said cycle can be substituted by 1 to 5 $R_1$ radicals, preferably with 1 to 3, $R_1$ having the meanings mentioned hereinabove in Claim 20.

**25.** A process according to one of Claims 18 and 19, characterised in that the carboxylic acid or derivative corresponds to general formula (II), wherein the R grouping represents a straight or branched acyclic aliphatic residue preferably having 1 to 12 carbon atoms, either in saturated form or comprising one or several unsaturations on the chain,

usually 1 to 3 unsaturations, which can be single or conjugate double bonds or triple bonds; the hydrocarbon chain can possibly be:

- interrupted by one of the following groups:
  -O-,-CO-,

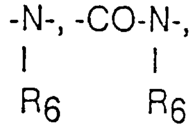

  -S-, $-SO_2-$
  in which formulae, $R_6$ represents hydrogen or a straight or branched alkyl radical with 1 to 4 carbon atoms, preferably a methyl or ethyl radical,
- and/or carrying one of the following substituents:
  -OH, $-COOR_5$, -CHO, $-NO_2$, -CN, $-NH_2$, -SH, -X, $-CF_3$,
  in which formulae, $R_5$ has the meaning given hereinabove in Claim 20.

**26.** A process according to one of Claims 18 and 19, characterised in that the carboxylic acid or derivative corresponds to general formula (II), wherein the R grouping corresponds to the following formula:

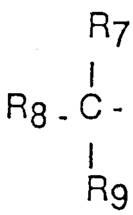

(IV)

wherein $R_7$, $R_8$ and $R_9$ which can be the same or different are selected from a hydrogen atom, a straight or branched alkyl radical containing 1 to 10 carbon atoms, a straight or branched alkenyl radical containing 1 to 10 carbon atoms, a straight or branched alkoxy radical containing 1 to 10 carbon atoms, a hydroxyl group, an amine function or a halogen atom or a $-CF_3$ group.

**27.** A process according to Claim 26, characterised in that the carboxylic acid or derivative corresponds to the general formula (II) in which the R grouping corresponds to formula (IV) in which one of the 3 groupings $R_7$, $R_8$ and $R_9$ has a carbon atom which forms a double bond with the carbonyl grouping of the carboxylic acid, ester or anhydride.

**28.** A process according to one of Claims 18 and 19, characterised in that the carboxylic acid or derivative corresponds to the general formula (II), wherein the R grouping represents a straight or branched, saturated or unsaturated acyclic aliphatic residue which can optionally carry a cyclic substituent, said acyclic aliphatic residue being able to be linked to the cycle by a valency bond or by one of the following groups:
-O-,-CO-,

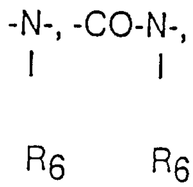

-S-, $-SO_2-$
in which formulae, $R_6$ has the meaning given hereinabove in Claim 20.

**29.** A process according to one of Claims 18 and 19, characterised in that the carboxylic acid or derivative corresponds to the general formula (II), wherein the R grouping represents a heterocyclic residue which may or may not be saturated and which comprises, in particular, 5 or 6 atoms in the cycle, including 1 or 2 heteroatoms such as nitrogen, sulphur or oxygen atoms; the carbon atoms of the heterocycle can possibly be substituted wholly or only partly by radicals $R_1$, $R_1$ having the meanings mentioned hereinabove in Claim 20, or the R grouping represents a polycyclic heterocyclic residue.

**30.** A process according to one of Claims 1 to 29, characterised in that the initial substrate is any mono- or polycarboxylic carboxylic acid, such as the saturated or unsaturated aliphatic acids; monocyclic or polycyclic, saturated, unsaturated or aromatic carbocyclics or heterocyclics; saturated or unsaturated aliphatics carrying a cyclic substituent such as a saturated, unsaturated or aromatic carbocyclic or heterocyclic cycle.

**31.** A process according to Claims 1 to 30, characterised in that the carboxylic acid or derivative corresponding to general formula (II) is selected from:

- saturated, aliphatic, monocarboxylic acids,
- saturated, aliphatic, dicarboxylic acids,
- unsaturated, aliphatic, dicarboxylic or monocarboxylic acids.
- saturated or unsaturated, carbocyclic, carboxylic acids,
- heterocyclic, carboxylic acids,
- aromatic, carbocyclic, carboxylic acids,
- saturated or unsaturated, aryl aliphatic, carboxylic acids,
- halogenated aliphatic or aromatic, carboxylic acids,
- aliphatic, cycloaliphatic or arylaliphatic hydroxy acids,
- hydroxybenzoic acids,
- alkoxy and phenoxy acids,
- oxo acids,
- acyloxy acids,
- amido acids,
- optionally N-protected amino acids.

**32.** A process according to one of Claims 1 to 31, characterised in that the carboxylic acid or derivative corresponding to general formula (II) is selected from:

. saturated aliphatic monocarboxylic acids, such as formic, acetic, propionic, butyric, isobutyric, valeric, isovaleric, pivalic, lauric, myristic, palmitic, stearic acids,

- saturated aliphatic dicarboxylic acids, such as oxalic, malonic, succinic, glutaric, adipic, pimelic, suberic, azelaic, sebacic acids,
- unsaturated aliphatic monocarboxylic or dicarboxylic acids, such as acrylic, propiolic, methacrylic, crotonic, isocrotonic, senecioic, tiglic, oleic, maleic, fumaric, citraconic, mesaconic acids,
- saturated or unsaturated carbocyclic carboxylic acids, such as camphoric acid, chrysanthemic acid,
- heterocyclic carboxylic acids, such as furan carboxylic, thiophene carboxylic, pyrrole carboxylic, pyrazine carboxylic, nicotinic, isonicotinic, picolinic acids,
- aromatic carbocyclic carboxylic acids, such as benzoic, phthalic, isophthalic, terephthalic, naphthalene carboxylic, toluic acids,
- saturated arylaliphatic carboxylic acids, such as, in particular, the arylpropionics such as 2-phenyl propionic acid, [(2-butyl)-4 phenyl]-2 propionic acid, (3-benzoyl phenyl)-2 propionic acid, (6-methoxy 2-naphtyl)-2 propionic acid or unsaturated acids, such as 2-phenyl propenoic acid, cinnamic acid,
- halogenated aliphatic or aromatic, carboxylic acids such as monofluoroacetic, difluoroacetic, monochloroacetic, dichloroacetic, trichloroacetic, monochloropropionic, $\alpha$-bromopropionic, $\alpha$-bromobutyric, trifluoroacetic, monofluoro-o-benzoic, monofluoro-m-benzoic, monofluoro-p-benzoic, difluoro-2,3-benzoic, difluoro-2,4-benzoic, difluoro-2,5-benzoic, difluoro-3,4-benzoic, trifluoro-2,3,6-benzoic, trifluoro-2,4,5-benzoic, tetrafluoro-2,3,4,5-benzoic, pentafluorobenzoic, $\alpha,\alpha,\alpha$-trifluoro-o-toluic, $\alpha,\alpha,\alpha$-trifluoro-m-toluic, $\alpha,\alpha,\alpha$-trifluoro-p-toluic,monochloro-o-benzoic,monochloro-m-benzoic,monochloro-p-benzoic, dichloro-2,3-benzoic, dichloro-2,4-benzoic, dichloro-2,5-benzoic, dichloro-2,6-benzoic, dichloro-3,4-benzoic, dichloro-3,5-benzoic, trichloro-2,3,5-benzoic, trichloro-2,3,6-benzoic, 2-chloro-4,5-fluorobenzoic, 3-chloro-2,4,5-trifluorobenzoic, monobromo-o-benzoic, monobromo-m-benzoic and monobromo-p-benzoic acids.

- arylaliphatic cycloaliphatic aliphatic hydroxy acids, such as glycolic acid, lactic acid, glyceric acid, 2-hydroxy butanoic acid, 3-hydroxy butanoic acid, 2-methyl lactic acid, 2-hydroxy 4-methylthio butanoic acid, tartronic acid, malic acid, tartric acid, 1-hydroxy cyclopropane carboxylic acid, 2-hydroxy phenylpropanoic acid, 2-hydroxy cinnamic acid, 3-hydroxy cinnamic acid, 4-hydroxy cinnamic acid,
- the following hydroxybenzoic acids: 2-hydroxy benzoic acid (salicylic acid), 3-hydroxy benzoic acid, 4-hydroxy benzoic acid, 3-methyl salicylic acid, 4-methyl salicylic acid, 5-methyl salicylic acid, 3-hydroxy 4-methyl benzoic acid, 3-methoxy salicylic acid, 4-methoxy salicylic acid, 5-methoxy salicylic acid, 3-hydroxy 4-methoxy benzoic acid (isovanillic acid), 4-hydroxy 3-methoxy benzoic acid (vanillic acid), 3-hydroxy 4,5-dimethoxy benzoic acid, 4-hydroxy 3,5-dimethoxy benzoic acid (syringic acid), 5-hydroxy isophthalic acid, 3-amino salicylic acid, 4-amino salicylic acid, 5-amino salicylic acid, 3-hydroxy 2-amino benzoic acid, 3-nitro salicylic acid, 3-hydroxy 4-nitro benzoic acid, 4-hydroxy 3-nitro benzoic acid, 3-hydroxy 4-methyl 2-nitro benzoic acid, 3,5-diiodo salicylic acid, 2,3-dihydroxy benzoic acid, 2,4-dihydroxy benzoic acid, 2,5-dihydroxy benzoic acid, 2,6-dihydroxy benzoic acid, 3,4-dihydroxy benzoic acid (protocatechic acid), 3,5-dihydroxy benzoic acid, 3,5-dihydroxy 4-methyl benzoic acid, 2,3,4-trihydroxy benzoic acid, 2,4,6-trihydroxy benzoic acid, 3,4,5-trihydroxy benzoic acid,
- alkoxy and phenoxy acids, such as methoxyacetic acid, phenoxyacetic, 2,4-dichloro phenoxy acetic, phenoxy propionic, 2,4-dichloro phenoxy propionic, p-hydroxy phenoxy propionic, m-chlorophenoxy propionic, 4-phenoxy benzoic acid, (4-carboxy 4-phenoxy) benzoic acid, piperonylic acid,
- oxo-acids, such as 2-acetyl benzoic acid, 4-acetyl benzoic acid, 2-benzoyl benzoic acid, 4-benzoyl benzoic acid,
- acyloxy acids, such as 3-benzoyloxy propionic acid, 2-acetoxy benzoic acid, 4-acetoxy benzoic acid,
- amido acids, such as 2-acetamido acrylic acid, 2-acetamido benzoic acid, 3-acetamido benzoic acid, 4-N-acetamido benzoic acid,
- amine acids, possibly N-protected by a protector group such as the following groups: alkyl (acetyl, benzoyl), BOC (butyloxycarbonyl), CBZ (carbobenzoxy), FMOC (9-fluorenyl methoxycarbonyl), MSOC (2-methane sulphenyl ethoxy carbonyl).

**33.** A process according to Claim 32, characterised in that the carboxylic acid or derivative corresponding to general formula (II) is selected from:

- saturated or unsaturated carboxylic or dicarboxylic acids,
- salicylic acid and 4-hydroxy benzoic acid.
- acetic acid, propionic acid and their derivatives, substituted by a hydroxy group, halogen, phenyl, phenyloxy,
- benzoic acid and its derivatives substituted by an alkyl $C_1$-$C_4$ group, acetoxy, acetamido, hydroxy, methoxy, ethoxy,
- halogenated aliphatic or aromatic carboxylic acids,
- nicotinic acid.

**34.** A process according to one of Claims 1 to 33, characterised in that the aldehyde prepared corresponds, more particularly, to the general formula (V):

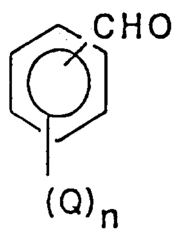

(V)

wherein:

- n is equal to 0,1,2 or 3,
- Q represents one of the following groups or functions:

  . a hydrogen atom,
  . a straight or branched alkyl radical, with 1 to 4 carbon atoms,
  . a straight or branched alkoxy radical with 1 to 4 carbon atoms,

. a methylene or ethylene dioxy radical,
. a -OH group,
. a -CHO group,
. a $NH_2$ group,
. a phenyl radical,
. a halogen atom,
. a $CF_3$ group.

**35.** A process according to one of Claims 1 to 34, characterised in that the aldehyde prepared is more particularly 3,4-difluorobenzaldehyde, 4-chlorobenzaldehyde, salicylic aldehyde, 3-hydroxybenzaldehyde, 4-hydroxybenzaldehyde, vanillin, veratraldehyde, p-anisaldehyde, sinnamic aldehyde, piperonal, fluoral, acetic aldehyde, prenal or citral.

**36.** Use of the salicylic aldehyde obtained according to the preparations process described in one of Claims 1 to 33 as a coumarin production intermediate.

**37.** A bimetallic catalyst comprising tin and ruthenium, free of boron, optionally on a support, characterised in that it comprises ruthenium and tin used in quantities such that the tin/ruthenium molar ratio is at least 2, preferably between 2 and 10 inclusive, and still more preferably between 2 and 6 inclusive.

**38.** A catalyst according to Claim 37, characterised in that it is prepared by depositing, in known manner, metallic elements on the support, and by then subjecting the contact mass to reduction by means of hydrogen.

**39.** A catalyst according to any one of Claims 37 and 38, characterised in that the ruthenium represents between 0.1% and 50% approximately of the weight of the catalyst.

**40.** A catalyst according to Claim 39, characterised in that the ruthenium represents from 10 to 50% by weight of the catalyst when it is in mass form, and from 0.1 to 20%, preferably from 0.5 to 3% by weight of the catalyst when it is supported.

**41.** A catalyst according to Claim 37, characterised in that it is supported and in that the support is selected from oxides of metals, preferably the oxides of aluminium and/or silicon, carbons, acetylene black and resins.

**42.** A catalyst according to one of Claims 37 to 41, characterised in that the ruthenium is introduced in the form of metallic ruthenium, ruthenium chloride III, ruthenium chloride IV, ruthenium pentafluoride, ruthenium oxide II, ruthenium oxide IV, ruthenium oxychloride with ammonia, ruthenium acetate, preferably ruthenium chloride III.

**43.** A catalyst according to one of Claims 37 to 42, characterised in that the tin is introduced in the form of metallic tin, oxides, chlorides, nitrates, carboxylates, alcoholates of tin or organometallic compounds in which the tin is linked to a hydrogen atom and/or alkyl radicals which preferably have from 1 to 4 carbon atoms, preferably tin chloride II, tin chloride IV, tin acetate II, tin octoate II, tin ethylhexanoate.

**Patentansprüche**

**1.** Verfahren zur Herstellung von Aldehyden und ihrer Derivate mittels Wasserstoffreduktion von Carbonsäuren, Carbonsäureestern oder Carbonsäureanhydriden, dadurch gekennzeichnet, daß das Verfahren in der Gasphase in Gegenwart eines bimetallischen Katalysators vom Ruthenium/Zinn-Typ durchgeführt wird.

**2.** Verfahren nach Anspruch 1, dadurch gekennzeichnet, daß der Katalysator ein Ruthenium/Zinn/Bor-Katalysator ist.

**3.** Verfahren nach Anspruch 2, dadurch gekennzeichnet, daß der Borgehalt unter 1 %, vorzugsweise bei etwa 0,5 %, liegt.

**4.** Verfahren nach Anspruch 1, dadurch gekennzeichnet, daß der Katalysator ein borfreier bimetallischer Ruthenium/Zinn-Katalysator ist.

**5.** Verfahren nach Anspruch 1, dadurch gekennzeichnet, daß der Katalysator so beschaffen ist, daß das Sn/Ru-Molverhältnis zwischen eingesetztem Zinn und Ruthenium zwischen 1 und 10, vorzugsweise zwischen 2 und 6, liegt.

6. Verfahren nach einem der Ansprüche 1 bis 5, dadurch gekennzeichnet, daß das Ruthenium etwa 0,1 % bis 50 % des Katalysatorgewichts darstellt.

7. Verfahren nach Anspruch 6, dadurch gekennzeichnet, daß das Ruthenium 10 bis 50 % des Katalysatorgewichts darstellt, wenn er in Masse vorliegt, und 0,1 bis 20 %, vorzugsweise 0,5 bis 3 %, des Katalysatorgewichts darstellt, wenn er als Trägerkatalysator vorliegt.

8. Verfahren nach einem der Ansprüche 1 bis 7, dadurch gekennzeichnet, daß man einen Trägerkatalysator verwendet.

9. Verfahren nach Anspruch 8, dadurch gekennzeichnet, daß der Träger aus Metalloxiden, Kohlen und Harzen ausgewählt ist.

10. Verfahren nach Anspruch 9, dadurch gekennzeichnet, daß der Träger Aluminium- und/oder Siliciumoxid ist.

11. Verfahren nach einem der Ansprüche 8 bis 10, dadurch gekennzeichnet, daß der Träger eine spezifische Oberfläche (BET) aufweist, die zwischen 50 und 100 $m^2/g$ ausgewählt ist, wenn der Rutheniumgehalt des Katalysators 0,1 bis 1 % beträgt.

12. Verfahren nach Anspruch 1, dadurch gekennzeichnet, daß man bei einer Temperatur zwischen 150 °C und 500 °C, vorzugsweise zwischen 200 °C und 400 °C, verfährt.

13. Verfahren nach einem der Ansprüche 1 bis 12, dadurch gekennzeichnet, daß man den Katalysator zuvor durch starkes Anheben der Temperatur aktiviert, vorzugsweise durch Anheben auf Temperaturen von etwa 500 °C, insbesondere von 450 °C, wobei die Aktivierung vorzugsweise unter Wasserstoffstrom durchgeführt wird.

14. Verfahren nach Anspruch 1, dadurch gekennzeichnet, daß bei der Verwendung von 1 ml des Katalysators der Wasserstoff mit einem Durchsatz zwischen 0,1 und 10 Litern pro Stunde und die Säure, der Ester oder das Anhydrid mit einem Flüssigdurchsatz von höchstens 10 ml/h, vorzugsweise zwischen 0,5 und 5 ml/h, injiziert werden.

15. Verfahren nach Anspruch 1, dadurch gekennzeichnet, daß die Carbonsäure, der Ester oder das Anhydrid direkt in gasförmiger Form injiziert werden.

16. Verfahren nach Anspruch 1, dadurch gekennzeichnet, daß die Carbonsäure, der Ester oder das Anhydrid in flüssiger Form in einem inerten Solvens injiziert werden.

17. Verfahren nach Anspruch 1, dadurch gekennzeichnet, daß der Wasserstoff bei Atmosphärendruck oder unter leichtem Druck, gegebenenfalls in einem Inertgas verdünnt, injiziert wird.

18. Verfahren nach einem der Ansprüche 1 bis 17 zur Herstellung von Aldehyden der allgemeinen Formel

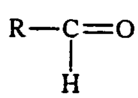 (I)

in der R ein Wasserstoffatom oder einen gegebenenfalls substituierten Kohlenwasserstoffrest mit 1 bis 40 Kohlenstoffatomen darstellt, der ein gesättigter oder ungesättigter, linearer oder verzweigter, acyclischer aliphatischer Rest sein kann; einen monocyclischen oder polycyclischen, gesättigten, ungesättigten oder aromatischen, carbocyclischen oder heterocyclischen Rest durch Reduktion von Estern, Anhydriden oder Säuren der Formel

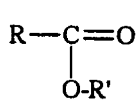 (II)

in der

- R wie zuvor definiert ist,
- R' folgendes darstellt:

  - eine wie zuvor definierte Gruppe R,
  - eine Gruppe R"-C=O, in der R" die für R angegebene Bedeutung hat,
  - die beiden Gruppen R und R" können miteinander verbunden sein, um einen gesättigten oder ungesättigten Ring mit 5 bis 7 Atomen zu bilden, der die Anhydridfunktion enthält,
  - die beiden Gruppen R und R" können mittels zweier vicinaler Atome zusammen eine Brücke eines orthokondensierten bicyclischen Systems bilden.

**19.** Verfahren nach Anspruch 18, dadurch gekennzeichnet, daß die Carbonsäure oder ihr Derivat der Formel (II) entspricht, in der die Gruppe R einen gegebenenfalls substituierten Kohlenwasserstoffrest mit 1 bis 20 Kohlenstoffatomen darstellt.

**20.** Verfahren nach einem der Ansprüche 18 und 19, dadurch gekennzeichnet, daß die Carbonsäure oder ihr Derivat der Formel (II) entspricht, in der die Gruppe R vorzugsweise einen aromatischen Kohlenwasserstoffrest, insbesondere einen benzolischen Rest, darstellt, der der allgemeinen Formel (III) entspricht

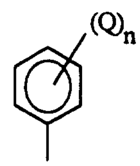

(III)

wobei in der besagten Formel (III)

- n eine ganze Zahl zwischen 0 und 5, vorzugsweise zwischen 0 und 3, ist,
- Q $R_1$ oder eine der folgenden Gruppen oder Funktionen darstellt:

  - einen linearen oder verzweigten Alkylrest mit 1 bis 6 Kohlenstoffatomen, vorzugsweise mit 1 bis 4 Kohlenstoffatomen, wie z.B. Methyl, Ethyl, Propyl, Isopropyl, Butyl, Isobutyl, sek.-Butyl, tert.-Butyl,
  - einen linearen oder verzweigten Alkenylrest mit 2 bis 6 Kohlenstoffatomen, vorzugsweise mit 2 bis 4 Kohlenstoffatomen, wie z.B. Vinyl oder Allyl,
  - einen linearen oder verzweigten Alkoxyrest mit 1 bis 6 Kohlenstoffatomen, vorzugsweise 1 bis 4 Kohlenstoffatomen, wie z.B. ein Methoxy-, Ethoxy-, Propoxy-, Isopropoxy- oder Butoxyrest,
  - eine Acylgruppe mit 2 bis 6 Kohlenstoffatomen,
  - einen Rest der Formel

$$-R_2-OH$$

$$-R_2-COOR_5$$

$$-R_2-CHO$$

$$-R_2-NO_2$$

$$-R_2-CN$$

$$-R_2-(NR_5)_2$$

$$-R_2-CO-(NR_5)_2$$

$-R_2-SH$

$-R_2-X$

$-R_2-CF_3$

wobei in diesen Formeln $R_2$ eine Valenzbindung oder einen gesättigten oder ungesättigten, linearen oder verzweigten, divalenten Kohlenwasserstoffrest mit 1 bis 6 Kohlenstoffatomen darstellt wie z.B. Methylen, Ethylen, Propylen, Isopropylen, Isopropyliden; $R_5$ ein Wasserstoffatom oder einen linearen oder verzweigten Alkylrest mit 1 bis 6 Kohlenstoffatomen darstellt; X ein Halogenatom, vorzugsweise ein Chlor-, Brom- oder Fluoratom, symbolisiert.

- Q $R_3$ oder einen der folgenden komplexeren Reste darstellt:

  • einen Rest

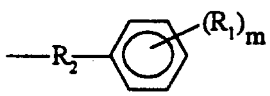

in der $R_1$ und $R_2$ die zuvor angegebene Bedeutung haben und m eine ganze Zahl zwischen 0 und 5, vorzugsweise zwischen 0 und 3, ist,

  • einen Rest $R_2$-A-$R_4$, in der $R_2$ die zuvor angegebene Bedeutung hat und $R_4$ einen linearen oder verzweigten Alkylrest mit 1 bis 6 Kohlenstoffatomen, vorzugsweise mit 1 bis 4 Kohlenstoffatomen, darstellt oder einen Rest der Formel

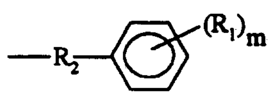

und A eine der folgenden Gruppierungen bedeutet:
-O-,-CO-,

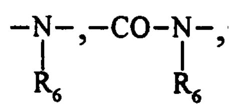

-S-,-$SO_2$-
wobei in diesen Formeln $R_6$ ein Wasserstoffatom oder einen linearen oder verzweigten Alkylrest mit 1 bis 4 Kohlenstoffatomen, vorzugsweise einen Methyl- oder Ethylnest, darstellt.

**21.** Verfahren nach Anspruch 20, dadurch gekennzeichnet, daß die Carbonsäure oder ihr Derivat der Formel (II) entspricht, in der die Gruppe R einen aromatischen Kohlenwasserstoffrest darstellt, der der allgemeinen Formel (III) entspricht, in der n größer als 1 ist, die Reste Q identisch oder verschieden sein können und zwei aufeinanderfolgende Kohlenstoffatome des Benzolrings miteinander über eine Ketalverbrückung verbunden sein können wie z.B. außerhalb des Kerns befindliche Dioxymethylen- oder Dioxyethylenreste.

**22.** Verfahren nach Anspruch 20, dadurch gekennzeichnet, daß die Carbonsäure oder ihr Derivat der allgemeinen Formel (II) entspricht, in der die Gruppe R einen aromatischen Rest darstellt, der der allgemeinen Formel (III) entspricht, in der

- n 0, 1, 2 oder 3 ist,

- Q eine der folgenden Gruppen oder Funktionen darstellt:

  • ein Wasserstoffatom,
  • einen linearen oder verzweigten Alkylrest mit 1 bis 4 Kohlenstoffatomen,
  • einen linearen oder verzweigten Alkoxyrest mit 1 bis 4 Kohlenstoffatomen,
  • ein Dioxymethylen- oder Dioxyethylenrest,
  • eine OH-Gruppe,
  • eine CHO-Gruppe,
  • eine $NH_2$-Gruppe,
  • einen Phenylrest,
  • einen Halogenatom
  • eine $CF_3$-Gruppe.

**23.** Verfahren nach einem der Ansprüche 18 und 19, dadurch gekennzeichnet, daß die Carbonsäure oder ihr Derivat der allgemeinen Formel (II) entspricht, in der die Gruppe R einen Naphthalinrest darstellt, wobei die Ringe mit 1 bis 4 Resten $R_1$, vorzugsweise 1 bis 3 Resten $R_1$, substituiert sein können, wobei $R_1$ die zuvor in Anspruch 20 genannten Bedeutungen hat.

**24.** Verfahren nach einem der Ansprüche 18 und 19, dadurch gekennzeichnet, daß die Carbonsäure oder ihr Derivat der allgemeinen Formel (II) entspricht, in der die Gruppe R einen gesättigten carbocyclischen Rest darstellt oder 1 oder 2 ungesättigte Bindungen im Ring enthält, wobei der im allgemeinen 3 bis 7 Kohlenstoffatome, vorzugsweise 6 Kohlenstoffatome, enthaltende Ring mit 1 bis 5 Resten $R_1$, vorzugsweise mit 1 bis 3 Resten $R_1$, substituiert sein kann, wobei $R_1$ die zuvor in Anspruch 20 aufgeführten Bedeutungen hat.

**25.** Verfahren nach einem der Ansprüche 18 und 19, dadurch gekennzeichnet, daß die Carbonsäure oder ihr Derivat der allgemeinen Formel (II) entspricht, in der die Gruppe R einen linearen oder verzweigten acyclischen aliphatischen Rest mit vorzugsweise 1 bis 12 Kohlenstoffatomen darstellt, der gesättigt sein kann oder eine oder mehrere ungesättigte Bindungen in der Kette enthalten kann, im allgemeinen 1 bis 3 ungesättigte Bindungen, die einfache oder konjugierte Doppelbindungen oder Dreifachbindungen sein können; die Kohlenwasserstoffkette kann gegebenenfalls

- von einer der folgenden Gruppierungen unterbrochen sein:
  -O-,-CO-,

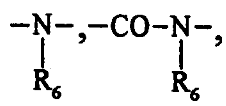

  -S-,-$SO_2$-
  wobei in diesen Formeln $R_6$ ein Wasserstoffatom oder ein linearer oder verzweigter Alkylrest mit 1 bis 4 Kohlenstoffatomen, vorzugsweise ein Methyl- oder Ethylrest, sein kann
- und/oder Träger einer der folgenden Substituenten sein kann
  -OH, -$COOR_5$, -CHO, -$NO_2$, -CN, $NH_2$, -SH, -X, -$CF_3$,
  wobei in diesen Formeln $R_5$ die zuvor in Anspruch 20 angegebene Bedeutung hat.

**26.** Verfahren nach einem der Ansprüche 18 und 19, dadurch gekennzeichnet, daß die Carbonsäure oder ihr Derivat der allgemeinen Formel (II) entspricht, in der die Gruppe R der folgenden Formel entspricht

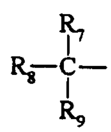 (IV)

in der $R_7$, $R_8$ und $R_9$, identisch oder verschieden, ausgewählt sind aus einem Wasserstoffatom, einem linearen oder verzweigten Alkylrest mit 1 bis 10 Kohlenstoffatomen, einem linearen oder verzweigten Alkenylrest mit 1 bis 10

Kohlenstoffatomen, einem linearen oder verzweigten Alkoxyrest mit 1 bis 10 Kohlenstoffatomen, einer Hydroxylgruppe, einer Aminfunktion, einem Halogenatom oder einer $CF_3$-Gruppe.

**27.** Verfahren nach Anspruch 26, dadurch gekennzeichnet, daß die Carbonsäure oder ihr Derivat der allgemeinen Formel (II) entspricht, in der die Gruppe R der Formel (IV) entspricht, in der eine der drei Gruppen $R_7$, $R_8$ und $R_9$ ein Kohlenstoffatom aufweist, das eine konjugierte Doppelbindung mit der Carbonylgruppe der Säure, des Esters oder des Carbonsäureanhydrids bildet.

**28.** Verfahren nach einem der Ansprüche 18 und 19, dadurch gekennzeichnet, daß die Carbonsäure oder ihr Derivat der allgemeinen Formel (II) entspricht, in der die Gruppe R einen linearen oder verzweigten, gesättigten oder ungesättigten, acyclischen aliphatischen Rest darstellt, der gegebenenfalls Träger eines cyclischen Substituenten sein kann, wobei der acyclische aliphatische Rest mit einem Ring über eine Valenzbindung oder über die folgenden Gruppierungen verbunden sein kann
-O-,-CO-,

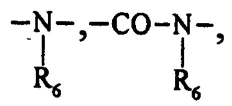

-S-,-$SO_2$-
wobei in diesen Formeln $R_6$ die zuvor in Anspruch 20 angegebene Bedeutung hat.

**29.** Verfahren nach einem der Ansprüche 18 und 19, dadurch gekennzeichnet, daß die Carbonsäure oder ihr Derivat der allgemeinen Formel (II) entspricht, in der die Gruppe R einen heterocyclischen gesättigten oder ungesättigten Rest darstellt, der insbesondere 5 oder 6 Atome im Ring trägt, von denen 1 oder 2 Heteroatome sind wie z.B. Stickstoff, Schwefel oder Sauerstoff; die Atome des Kohlenstoffs des Heterozyklus können gegebenenfalls, in ihrer Gesamtheit oder nur teilweise, mit den Resten $R_1$ substituiert sein, wobei $R_1$ dieselben Bedeutungen wie zuvor in Anspruch 20 angegeben haben oder die Gruppe R einen polycyclischen heterocyclischen Rest darstellt.

**30.** Verfahren nach einem der Ansprüche 1 bis 29, dadurch gekennzeichnet, daß die Ausgangsverbindung jede beliebige Carbonsäure mit einer oder mehreren Carboxylfunktionen ist wie z.B. gesättigte oder ungesättigte aliphatische Säuren; carbocyclische oder heterocyclische, gesättigte, ungesättigte oder aromatische, monocyclische oder polycyclische Säuren; gesättigte oder ungesättigte aliphatische Säuren, die einen cyclischen Substituenten tragen wie z.B. einen gesättigten, ungesättigten oder aromatischen, carbocyclischen oder heterocyclischen Ring.

**31.** Verfahren nach einem der Ansprüche 1 bis 30, dadurch gekennzeichnet, daß die Carbonsäure oder ihr Derivat der allgemeinen Formel (II) entspricht und ausgewählt ist aus

- gesättigten aliphatischen Monocarbonsäuren,
- gesättigten aliphatischen Dicarbonsäuren,
- ungesättigten aliphatischen Mono- oder Dicarbonsäuren,
- gesättigten oder ungesättigten carbocyclischen Carbonsäuren,
- heterocyclischen Carbonsäuren,
- aromatischen carbocyclischen Carbonsäuren,
- gesättigten oder ungesättigten arylaliphatischen Carbonsäuren,
- halogenierten aliphatischen oder aromatischen Carbonsäuren,
- aliphatischen, cycloaliphatischen und arylaliphatischen Hydroxysäuren,
- Hydroxybenzoesäuren,
- Alkoxy- und Phenoxysäuren,
- Oxosäuren,
- Acyloxysäuren,
- Amidosäuren,
- gegebenenfalls N-geschützten Aminosäuren.

**32.** Verfahren nach einem der Ansprüche 1 bis 31, dadurch gekennzeichnet, daß die Carbonsäure oder ihr Derivat der allgemeinen Formel (II) entspricht und ausgewählt ist aus

- gesättigten aliphatischen Monocarbonsäuren wie Ameisensäure, Essigsäure, Propionsäure, Buttersäure, Isobuttersäure, Valeriansäure, Isovaleriansäure, Pivalinsäure, Laurinsäure, Myristinsäure, Palmitinsäure und Stearinsäure,
- gesättigten aliphatischen Dicarbonsäuren wie z.B. Oxalsäure, Malonsäure, Bernsteinsäure, Glutarsäure, Adipinsäure, Pimelinsäure, Suberinsäure, Azelainsäure und Sebacinsäure,
- ungesättigten aliphatischen Mono- oder Dicarbonsäuren wie z.B. Acrylsäure, Propiolsäure, Methacrylsäure, Crotonsäure, Isocrotonsäure, Seneciosäure, Tiglinsäure, Oleinsäure, Maleinsäure, Fumarsäure, Citraconsäure und Mesaconsäure,
- gesättigten oder ungesättigten carbocyclischen Carbonsäuren wie z.B. Camphersäure und Chrysanthemumsäure,
- heterocyclischen Carbonsäuren wie z.B. Furancarbonsäuren, Thiophencarbonsäuren, Pyrrolcarbonsäuren, Pyrazincarbonsäuren, Nicotinsäure, Isonicotinsäure und Picolinsäure,
- aromatischen carbocyclischen Carbonsäuren wie z.B. Benzoesäure, Phthalsäure, Isophthalsäure, Terephthalsäure, Naphthalincarbonsäuren und Toluylsäuren,
- gesättigten arylaliphatischen Carbonsäuren wie z.B. insbesondere Arylpropionsäuren wie z.B. 2-Phenylpropionsäure, 2-[4-(2-Butyl)-phenyl]-propionsäure, 2-(3-Benzoylphenyl)-propionsäure, 2-(6-Methoxy-2-naphthyl)-propionsäure oder ungesättigten Säuren wie z.B. 2-Phenylpropensäure und Zimtsäure,
- halogenierten aliphatischen oder aromatischen Carbonsäuren wie Monofluoressigsäure, Difluoressigsäure, Monochloressigsäure, Dichloressigsäure, Trichloressigsäure, Monochlorpropionsäure, α-Brompropionsäure, α-Brombuttersäure, Trifluoressigsäure, Monofluor-o-benzoesäure, Monofluor-m-benzoesäure, Monofluor-p-benzoesäure, 2,3-Difluorbenzoesäure, 2,4-Difluorberzoesäure, 2,5-Difluorbenzoesäure, 3,4-Difluorbenzoesäure, 2,3,6-Trifluorbenzoesäure, 2,4,5-Trifluorbenzoesäure, 2,3,4,5-Tetrafluorbenzoesäure, Pentafluorbenzoesäure, α,α,α-Trifluor-o-toluylsäure, α,α,α-Trifluor-m-toluylsäure, α,α,α-Trifluor-p-toluylsäure, Monochlor-o-benzoesäure, Monochlor-m-benzoesäure, Monochlor-p-benzoesäure, 2,3-Dichlorbenzoesäure, 2,4-Dichlorbenzoesäure, 2,5-Dichlorbenzoesäure, 2,6-Dichlorbenzoesäure, 3,4-Dichlorbenzoesäure, 3,5-Dichloibenzoesäure, 2,3,5-Trichlorbenzoesäure, 2,3,6-Trichlorbenzoesäure, 2-Chlor-4,5-fluorbenzoesäure, 3-Chlor-2,4,5-trifluorbenzoesäure, Monobrom-o-benzoesäure, Monobrom-m-benzoesäure, Monobrom-p-benzoesäure,
- aliphatische cycloaliphatische und arylaliphatische Hydroxysäuren wie z.B. Glykolsäure, Milchsäure, Glycerinsäure, 2-Hydroxybutansäure, 3-Hydroxybutansäure, 2-Methylmilchsäure, 2-Hydroxy-4-methylthiobutansäure, Tartronsäure, Apfelsäure, Weinsäure, 1-Hydroxycyclopropancarbonsäure, 2-Hydroxyphenylpropansäure, 2-Hydroxyzimtsäure, 3-Hydroxyzimtsäure, 4-Hydroxyzimtsäure,
- den folgenden Hydroxybenzoesäuren: 2-Hydroxybenzoesäure (Salicylsäure), 3-Hydroxybenzoesäure, 4-Hydroxybenzoesäure, 3-Methylsalicylsäure, 4-Methylsalicylsäure, 5-Methylsalicylsäure, 3-Hydroxy-4-methylbenzoesäure, 3-Methoxysalicylsäure, 4-Methoxysalicylsäure, 5-Methoxysalicylsäure, 3-Hydroxy-4-methoxybenzoesäure (Isovanillinsäure), 4-Hydroxy-3-methoxybenzoesäure (Vanillinsäure), 3-Hydroxy-4,5-dimethoxybenzoesäure, 4-Hydroxy-3,5-dimethoxybenzoesäure (Syringinsäure), 5-Hydroxyisophthalsäure, 3-Aininosalicylsäure, 4-Aminosalicylsäure, 5-Aminosalicylsäure, 3-Hydroxy-2-aminobenzoesäure, 3-Nitrosalicylsäure, 3-Hydroxy-4-nitrobenzoesäure, 4-Hydroxy-3-nitrobenzoesäure, 3-Hydroxy-4-methyl-2-nitrobenzoesäure, 3,5-Diiodsalicylsäure, 2,3-Dihydroxybenzoesäure, 2,4-Dihydroxybenzoesäure, 2,5-Dihydroxybenzoesäure, 2,6-Dihydroxybenzoesäure, 3,4-Dihydroxybenzoesäure (Protocatechinsäure), 3,5-Dihydroxybenzoesäure, 3,5-Dihydroxy-4-methylbenzoesäure, 2,3,4-Trihydroxybenzoesäure, 2,4,6-Trihydroxybenzoesäure, 3,4,5-Trihydroxybenzoesäure,
- Alkoxy- und Phenoxysäuren wie z.B. Methoxyessigsäure, Phenoxyessigsäure, 2,4-Dichlorphenoxyessigsäure, Phenoxypropionsäure, 2,4-Dichlorphenoxypropionsäure, p-Hydroxyphenoxypropionsäure, m-chlorphenoxypropionsäure, 4-Phenoxybenzoesäure, (4-Carboxy-4-phenoxy)-benzoesäure, Piperonylsäure,
- Oxosäuren wie z.B. 2-Acerylbenzoesäure, 4-Acetylbenzoesäure, 2-Benzoylbenzoesäure, 4-Benzoylbenzoesäure,
- Acyloxysäuren wie z.B. 3-Benzoyloxypropionsäure, 2-Acetoxybenzoesäure, 4-Acetoxybenzoesäure,
- Amidosäuren wie z.B. 2-Acetamidoacrylsäure, 2-Acetamidobenzoesäure, 3-Acetamidobenzoesäure, 4-N-Acetamidobenzoesäure,
- Aminosäuren, die gegebenenfalls N-geschützt sind durch eine Schutzgruppe wie z.B. die folgenden Gruppen: Acyl (Acetyl, Benzoyl), BOC (Butyloxycarbonyl), CBZ (Carbobenzoxy), FMOC (9-Fluorenylmethoxycarbonyl), MSOC (2-Methansulfenylethoxycarbonyl).

**33.** Verfahren nach Anspruch 32, dadurch gekennzeichnet, daß die Carbonsäure oder ihr Derivat der allgemeinen Formel (II) entspricht und ausgewählt ist aus:

- gesättigten oder ungesättigten Carbon- oder Dicarbonsäuren,
- Salicylsäure und 4-Hydroxybenzoesäure,

- Essigsäure, Propionsäure und ihren Derivaten, die durch eine Hydroxy-, Halogen-, Phenyl- oder Phenyloxygruppe substituiert sind,
- Benzoesäure und ihren Derivaten, die durch eine $C_1$-$C_4$-Alkyl-, Acetoxy-, Acetamido-, Hydroxy-, Methoxy- oder Ethoxygruppe substituiert sind,
- halogenierte aliphatische oder aromatische Carbonsäuren,
- Nicotinsäure.

**34.** Verfahren nach einem der Ansprüche 1 bis 33, dadurch gekennzeichnet, daß der hergestellte Aldehyd insbesondere der allgemeinen Formel (V) entspricht

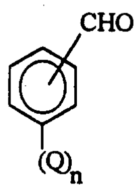

(V)

in der

- n 0, 1, 2 oder 3 ist,
- Q eine der folgenden Gruppen oder Funktionen darstellt:
  - ein Wasserstoffatom,
  - einen linearen oder verzweigten Alkylrest mit 1 bis 4 Kohlenstoffatomen,
  - einen linearen oder verzweigten Alkoxyrest mit 1 bis 4 Kohlenstoffatomen,
  - einen Dioxymethylen- oder Dioxyethylenrest,
  - eine OH-Gruppe,
  - eine CHO-Gruppe,
  - eine $NH_2$-Gruppe,
  - einen Phenylrest,
  - ein Halogenatom,
  - eine $CF_3$-Gruppe.

**35.** Verfahren nach einem der Ansprüche 1 bis 34, dadurch gekennzeichnet, daß der hergestellte Aldehyd insbesondere 3,4-Difluorbenzaldehyd, 4-Chlorbenzaldehyd, Salicylaldehyd, 3-Hydroxybenzaldehyd, 4-Hydroxybenzaldehyd, Vanillin, Veratrumaldehyd, p-Anisaldehyd, Zimtaldehyd, Piperonal, Fluoral, Acetaldehyd, Prenal oder Citral ist.

**36.** Verwendung des nach dem in den Ansprüchen 1 bis 33 beschriebenen Herstellungsverfahren erhaltenen Salicylaldehyds als Intermediat bei der Herstellung von Cumarin.

**37.** Bimetallischer Katalysator, enthaltend in borfreier Form Zinn und Ruthenium, gegebenenfalls auf einem Träger, dadurch gekennzeichnet, daß er eingesetztes Ruthenium und Zinn in solchen Mengen enthält, daß das Zinn/Ruthenium-Molverhältnis mindestens 2, vorzugsweise zwischen 2 und 10, insbesondere zwischen 2 und 6, ist.

**38.** Katalysator nach Anspruch 37, dadurch gekennzeichnet, daß er hergestellt ist durch Aufbringung von Metallelementen auf dem Träger in bekannter Weise und daß die Kontaktmasse dann einer Reduktion mittels Wasserstoff unterworfen wird.

**39.** Katalysator nach einem der Ansprüche 37 und 38, dadurch gekennzeichnet, daß das Ruthenium etwa 0,1 % bis 50 % des Katalysatorgewichts darstellt.

**40.** Katalysator nach Anspruch 39, dadurch gekennzeichnet, daß das Ruthenium 10 bis 50 % des Gewichts des Katalysators darstellt, wenn er in Masse vorliegt, und 0,1 bis 20 %, vorzugsweise 0,5 bis 3 %, des Katalysatorgewichts darstellt, wenn er als Trägerkatalysator vorliegt.

41. Katalysator nach Anspruch 37, dadurch gekennzeichnet, daß er als Trägerkatalysator vorliegt und daß der Träger ausgewählt ist aus Metalloxiden, vorzugsweise Aluminium- und/oder Siliciumoxid, Kohlen, Acetylenruß und Harzen

42. Katalysator nach einem der Ansprüche 37 bis 41, dadurch gekennzeichnet, daß das Ruthenium in Form von metallischem Ruthenium, Ruthenium-III-chlorid, Ruthenium-IV-chlorid, Rutheniumpentafluorid, Ruthenium-II-oxid, Ruthenium-IV-oxid, ammoniakalischem Rutheniumoxychlorid, Rutheniumacetat, vorzugsweise in Form von Ruthenium-III-chlorid, eingebracht wird.

43. Katalysator nach einem der Ansprüche 37 bis 42, dadurch gekennzeichnet, daß das Zinn eingebracht wird in Form von metallischem Zinn, Zinnoxiden, -chloriden, -nitraten, -carboxylaten und -alkoholaten oder in Form von organometallischen Verbindungen, in denen das Zinn an ein Wasserstoffatom und/oder an Alkylreste mit vorzugsweise 1 bis 4 Kohlenstoffatomen gebunden ist, vorzugsweise in Form von Zinn-II-chlorid, Zinn-IV-chlorid, Zinn-II-acetat, Zinn-II-octoat und Zinnethylhexanoat.